(19)

Europäisches
Patentamt
European
Patent Office
Office européen
des brevets

(11) **EP 4 385 999 A1**

(12) **EUROPEAN PATENT APPLICATION**

(43) Date of publication:
**19.06.2024 Bulletin 2024/25**

(21) Application number: **22213518.8**

(22) Date of filing: **14.12.2022**

(51) International Patent Classification (IPC):
***C07K 16/10*** *(2006.01)*

(52) Cooperative Patent Classification (CPC):
**C07K 16/1003;** A61K 2039/505; A61K 2039/507;
C07K 2317/21; C07K 2317/31; C07K 2317/33;
C07K 2317/35; C07K 2317/565; C07K 2317/76

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **ModiQuest B.V.
5349 AB  Oss (NL)**

(72) Inventor: **The designation of the inventor has not
yet been filed**

(74) Representative: **J A Kemp LLP
80 Turnmill Street
London EC1M 5QU (GB)**

Remarks:
The complete document including Reference
Table(s) and the Sequence Listing(s) can be
downloaded from the EPO website

(54) **ANTIBODIES**

(57)    The invention relates to antibodies and cocktails of antibodies useful for the prevention, treatment and/or diagnosis of coronavirus infections, and diseases and/or complications associated with coronavirus infections, including COVID-19.

EP 4 385 999 A1

**Description**

**Field of invention**

The invention relates to antibodies and cocktails of antibodies useful for the prevention, treatment and/or diagnosis of coronavirus infections, and diseases and/or complications associated with coronavirus infections, including COVID-19.

**Background of the invention**

The novel Coronavirus disease 2019 (COVID-19) is caused by a new severe acute respiratory syndrome coronavirus 2 (SARS-CoV-2). An infection with SARS-CoV-2 is initiated by the spike protein (S). This protein binds to the cellular receptor angiotensin-converting enzyme 2 (ACE2) during infection. The homotrimeric S protein is comprised of monomers which consist of a N-terminal S1 subunit responsible for receptor binding and a C-terminal S2 subunit responsible for membrane fusion. The receptor-binding domain (RBD) of S interacts with ACE2, and is a primary target for neutralizing antibodies. In patients infected with COVID-19 and vaccinated animals there is a dominance of RBD-directed antibodies in the neutralizing antibody response.

The RBD comprises a core structure, and a receptor-binding motif (RBM), which mediates direct contact with ACE2. The RBM contains antigenic sites that are capable of eliciting potent neutralizing antibodies. Several highly potent neutralizing monoclonal antibodies (mAbs) targeting the RBM have been isolated from COVID-19 patients. In addition, a number of core region-directed antibodies, cross-reactive to SARS-CoV, have also been identified. Despite its important critical function, the RBD domain of SARS- CoV-2 continuously evolves. Clear evidence is provided by the emergence of viral isolates with diverse amino acid changes in both the RBM and core regions of the RBD (Ou et al., Journal of Virology, 95(16): e00617-22 (2021)). Since the binding to ACE2 tolerates a substantial number of mutations (Starr et al., Cell, 182: 1295-1310.e20 (2020) and Yi et al., Cell. Mol. Immunol. 17: 621-630 (2020)), the concern of neutralization escape is raised. This might limit the therapeutic potential of neutralizing antibodies. A recent study, using a recombinant Vesicular Stomatitis Virus (VSV) that expresses S of SARS-CoV-2, demonstrated the rapid occurrence of escape mutants in the presence of RBD-specific single mAbs (Baum et al., Science 369: 1014-1018 (2020)).

Antibody cocktails are promising treatments that can prevent SARS-CoV-2 escape (Wang et al., J. Virol., 92 (2018); ter Meulen et al., PLos Med., 3: e237 (2006)). For instance it was shown that two antibodies that bound to non-overlapping epitopes on the receptor-binding domain (RBD) prevented viral escape (Ku et al., Nat. Commun., 12: 469 (2021)). Multiple antibody cocktails in clinical trials ( Baum et al., Science 369: 1014-1018 (2020), Zost et al., Nature, 584: 443-449 (2020), Du et al., Cell, 183: 1013-1023.e13 (2020) and Wu et al., Science, 368: 1274-1278 (2020)) are being evaluated for neutralization activities. Antibody cocktails and multispecific antibodies are described in PCT/EP2022/062777.

**Summary of the invention**

The inventors have generated a number of antibodies derived from patients infected with the SARS-CoV-2 virus. The antibodies were selected based on (i) binding to SARS-CoV-2 spike protein RBD, S1 or trimer; (ii) having high neutralization efficiency against SARS-CoV-2; (iii) having high binding affinity; and/or (iv) maintaining binding affinity for known variants of the SARS-CoV-2. Another advantage of the antibodies is that they are derived directly from convalescent (and vaccinated) patients having natural VH and VL pairings. Additionally, the antibodies were selected using the Omicron and Delta variants of the virus, and so are more effective against the viral strains that are currently most prevalent compared to previous antibodies raised against older variants of the virus.

The inventors further investigated the use of combinations of antibodies with characteristics (i) to (iv) which can cooperate with each other to prevent escape mutations. These mAbs bind to non-overlapping epitopes of the RBD and independently block the RBD-ACE2 interactions. These antibodies are thus particularly useful since they can both be used singly an in combination.

Accordingly, an aspect of the invention provides an antibody or an antigen-binding fragment thereof that binds to the spike protein of coronavirus SARS-CoV-2, wherein the antibody comprises a set of three heavy chain complementarity determining regions (CDRH1, CDRH2 and CDRH3) and three light chain complementarity determining regions (CDRL1, CDRL2 and CDRL3), wherein the set of CDRH1, CDRH2, CDRH3, CDRL1, CDRL2 and CDRL3 amino acid sequences are selected from: (a) SEQ ID NOs: 186, 187, 188, 190, 191 and 192 (Ab754); (b) SEQ ID NOs: 322, 323, 324, 326, 327 and 328 (Ab772); (c) SEQ ID NOs: 218, 219, 220, 222, 223 and 224 (Ab759); and (d) SEQ ID NOs: 250, 251, 252, 254, 255 and 256 (Ab763); (e) SEQ ID NOs: 234, 235, 236, 238, 239 and 240 (Ab761); (f) SEQ ID NOs: : 82, 83, 84, 86, 87 and 88 (Ab738); (g) SEQ ID NOs: 170, 171, 172, 174, 175 and 176 (Ab752); (h) SEQ ID NOs: 354, 355, 356, 358, 359 and 360 (Ab776); and (i) SEQ ID NOs: 410, 411, 412, 414, 415 and 416 (Ab783); (j) SEQ ID NOs: 458, 459, 460, 462, 463 and 464 (Ab789); (k) SEQ ID NOs: 434, 435, 436, 438, 439 and 440 (Ab786); (1) SEQ ID NOs: 138, 139, 140, 142, 143 and 144 (Ab748); (m) SEQ ID NOs: 242, 243, 244, 246, 247 and 248 (Ab762); (n) SEQ ID NOs: 98, 99, 100, 102, 103 and 104 (Ab740) (o) SEQ ID NOs: 306, 307, 308, 310, 311 and 312 (Ab770); and (p) SEQ ID NOs: 178, 179, 180, 182, 183 and 184 (Ab753).

In another aspect, the invention provides an antibody or an antigen-binding fragment thereof that binds to the spike protein of coronavirus SARS-CoV-2, wherein the antibody comprises a set of three heavy chain complementarity determining regions (CDRH1, CDRH2 and CDRH3) and/or three light chain complementarity determining regions (CDRL1, CDRL2 and CDRL3), of any of the antibodies shown in Table 3 herein.

The invention also provides multivalent or multispecific antibodies comprising the antigen binding regions of any of the antibodies above.

The invention also provides a combination of any two or more of the antibodies of Table 3 herein, or selected from antibodies (a) to (p), or (a) to (f) above.

The invention also provides a pharmaceutical composition comprising the antibody or combination of antibodies according to the invention, and optionally at least one pharmaceutically acceptable diluent or carrier.

The invention also provides the antibody, the combination of antibodies or the pharmaceutical composition according to the invention, for use in a method of treating or preventing a disease or a complication associated with coronavirus infection.

The invention also provides a method of treating a subject comprising administering a therapeutically effective amount of the antibody, the combination of antibodies or the pharmaceutical composition to the subject, or for treating or preventing a coronavirus infection, or a disease or complication associated with coronavirus infection.

The invention also provides the antibody, the combination of antibodies, or the pharmaceutical composition, for use in a method for treatment of a human or animal body by therapy, or for use in a method of treating or preventing a coronavirus infection, or a disease or complication associated with coronavirus infection.

The invention also provides the use of the antibody, the combination of antibodies or the pharmaceutical composition according to the invention in the manufacture of a medicament for treating a subject, or for treating or preventing a coronavirus infection, or a disease or complication associated with coronavirus infection.

The invention also provides a method of identifying the presence of coronavirus, or a protein or a protein fragment thereof, in a sample, comprising: (i) contacting the sample with an antibody or combination of antibodies of the invention; and (ii) detecting the presence or absence of an antibody-antigen complex, wherein the presence of the antibody-antigen complex indicates the presence of coronavirus, or a protein or a protein fragment thereof, in the sample. The invention also provides a method of treating or preventing coronavirus infection, or a disease or complication associated therewith, in a subject, comprising identifying the presence of coronavirus according to the method of the invention, and treating the subject with an anti-viral or an anti-inflammatory agent.

The invention also provides one or more polynucleotides that encode the antibody or combination of antibodies according to the invention, one or more vectors comprising the one or more polynucleotides, or a host cell comprising the one or more vectors.

The invention also provides a method for producing an antibody of the invention, the method comprising culturing a host cell of the invention and isolating the antibody from the culture.

The invention also provides the use of the antibody, the combination of antibodies, or the pharmaceutical composition according to the invention for preventing, treating and/or diagnosing coronavirus infection, or a disease or complication associated therewith.

The disclosure will now be described in more detail, by way of example and not limitation, and by reference to the accompanying drawings. Many equivalent modifications and variations will be apparent, to those skilled in the art when given this disclosure. Accordingly, the exemplary embodiments of the disclosure set forth are considered to be illustrative and not limiting. Various changes to the described embodiments may be made without departing from the scope of the invention, which is defined by the claims. All documents cited herein, whether supra or infra, are expressly incorporated by reference in their entirety.

## Brief description of the figures

Fig 1 - Serum ELISA. Absorbance is corrected by dividing the found value by a the absorbance measured from a control sample.

Fig 2 - Capture ELISA of B-cell wells. B-cells are described as Patient-Plate-Well. ELISA assay executed on the same day are grouped.

Fig 3 - Target ELISA B-cells. B-cells are described as Patient-Plate-Well.

Fig 4 - Neutralization B-cell wells. B-cells are described as Patient-Plate-Well. Samples denoted with a * are measured in single not duplo.

**Fig 5** - Binding assay of produced antibodies. ELISA assay using spike proteins of Delta and/or Omicron and BSA. An absorbance value above 0,5 a.u. is indicating a binding antibody. * denotes antibodies only tested on Omicron.

**Fig 6** - Omicron trimer spike protein neutralization assay of produced antibodies.

**Fig 7** - ELISA binding assay Omicron variant. Purified IgGs (10 nM in DPBS) were tested for binding to Omicron (B.1.1.529) Spike Trimer.

### Description of the Sequences

SEQ ID NOs: 1 to 2080 set forth the antibody sequences provided in Table 3.

SEQ ID NOs: 2081 sets forth the amino acid sequence of the spike protein of reference SARS-CoV-2 (2019-nCoV), Wuhan-Hu-1 strain.

SEQ ID NO: 2082 sets forth the DNA sequence of the variable light chain of Ab754.

SEQ ID NO: 2083 sets forth the DNA sequence of an exemplary expression plasmid encoding the kappa light chain of Ab754.

SEQ ID NO: 2084 sets forth the DNA sequence of the variable heavy chain of Ab754.

SEQ ID NO: 2085 sets forth the DNA sequence of an exemplary expression plasmid encoding the heavy chain of Ab754.

SEQ ID NO: 2086 sets forth the DNA sequence of the variable heavy chain of Ab772.

SEQ ID NO: 2087 sets forth the DNA sequence of the variable light chain of Ab772.

SEQ ID NO: 2088 sets forth the DNA sequence of the variable heavy chain of Ab759.

SEQ ID NO: 2089 sets forth the DNA sequence of the variable light chain of Ab759.

SEQ ID NO: 2090 sets forth the DNA sequence of an exemplary expression plasmid encoding the lambda light chain of Ab759.

SEQ ID NO: 2091 sets forth the DNA sequence of the variable heavy chain of Ab763.

SEQ ID NO: 2092 sets forth the DNA sequence of the variable light chain of Ab763.

SEQ ID NO: 2093 sets forth the DNA sequence of the variable heavy chain of Ab761.

SEQ ID NO: 2094 sets forth the DNA sequence of the variable light chain of Ab761.

SEQ ID NO: 2095 sets forth the sequence of the specific primer used for the sequencing of the pABS plasmids.

### Detailed description of the invention

B-cells from patients' blood samples were isolated and selected based on binding to a wild-type, Delta or Omicron spike protein. Selected B-cells were cultured and stimulated to produce antibodies. The produced antibodies were tested for binding and neutralization of the spike protein. B-cells clones which produced binding and/or neutralizing antibodies were sequenced. The sequencing results were analyzed and a selection of antibodies were produced as full-length IgG1 antibodies. The binding and neutralization of the antibodies were further analyzed. Of the best neutralizers the binding site was investigated using epitope binning.

A total of 63 patients were included in this study. The study population is an adult convalescent patient population that was infected with the SARS-CoV-2 virus, devoid of symptoms associated with an active SARS-CoV-2 infection, and/or had been vaccinated against SARS-CoV-2, and was suspected to have neutralizing antibodies against SARS-CoV-2 with a high affinity against SARS-CoV-2. Initially non-vaccinated patients were included. However, these patients did not show high SARS-CoV-2-specific B-cell counts. Therefore, it was decided to include convalescent patients who had been vaccinated. From these patients B-cells were sorted, cultured, the produced antibodies tested, and the best binders/ neutralizers sequenced. This resulted in 130 antibody sequences derived from the B-cells of 22 patients. The B-cells included were sorted using either Delta or Omicron Spike protein.

A first set of 62 antibodies, derived from B-cells selected for Delta spike, was produced in HEK293F cells and antibody yields were determined. The antibodies in cell culture medium were tested for binding to the Delta and/or Omicron spike proteins using ELISA. The neutralization of the antibodies was determined in a cell-based spike neutralization assay. A total of 16/62 antibodies showed both binding and neutralization. Using epitope binning these 16 lead antibodies were found to bind to three different epitopes.

The antibodies with the highest neutralization of the Omicron virus variant were Ab754, Ab759, Ab772, Ab763 and Ab761. These antibodies bind to two different epitopes. Another interesting candidate is Ab738 since it binds to a different epitope. Other interesting antibodies are Ab776, Ab789, Ab783, Ab786, Ab752, Ab762, Ab748 and Ab740. These antibodies also bind to both Omicron and Delta.

A second set of 83 antibodies are also similarly described herein. The antibody sequences are derived from B-cells selected for binding to either Delta or Omicron spike protein.

Antibodies of the invention

An antibody of the invention specifically binds to the spike protein of SAR-CoV-2, to the S1 subunit of the spike protein, or more specifically to the receptor binding domain (RBD).

In one embodiment, an antibody of the invention may comprise at least the six CDRs of an antibody listed in Table 3. In one embodiment, an antibody of the invention may comprise at least the six CDRs of an antibody having a designated used herein selected from Ab754, Ab759, Ab763, Ab761 and Ab772. In other embodiments an antibody of the invention may comprise at least the six CDRs of an antibody having a designated used herein selected from Ab754, Ab759, Ab763, Ab761, Ab772 and Ab738; or selected from Ab752, Ab776, Ab789, Ab783, Ab786, Ab762, Ab748 and Ab740; or selected from Ab754, Ab759, Ab763, Ab761, Ab772, Ab752, Ab776, Ab789, Ab783, Ab786, Ab762, Ab748 and Ab740; or selected from Ab759, Ab763, Ab761, Ab772, Ab738Ab752, Ab776, Ab789, Ab783, Ab786, Ab762, Ab748 and Ab740; or selected from Ab738Ab752, Ab776, Ab789, Ab783, Ab786, Ab762, Ab748 and Ab740; or selected from Ab770 and Ab753; or selected from Ab754, Ab759, Ab763, Ab761, Ab772 Ab770 and Ab753; or selected from Ab754, Ab759, Ab763, Ab761, Ab772, Ab738, Ab770 and Ab753; or selected from Ab754, Ab759, Ab763, Ab761, Ab772, Ab752, Ab776, Ab789, Ab783, Ab786, Ab762, Ab748, Ab740, Ab770 and Ab753; or selected from Ab754, Ab759, Ab763, Ab761, Ab772, Ab738, Ab752, Ab776, Ab789, Ab783, Ab786, Ab762, Ab748, Ab740, Ab770 and Ab753; or selected from Ab738, Ab770 and Ab753; or selected from Ab752, Ab776, Ab789, Ab783, Ab786, Ab762, Ab748, Ab740, Ab770 and Ab753; or selected from Ab738, Ab752, Ab776, Ab789, Ab783, Ab786, Ab762, Ab748, Ab740, Ab770 and Ab753, or any other combination of antibodies as described herein.

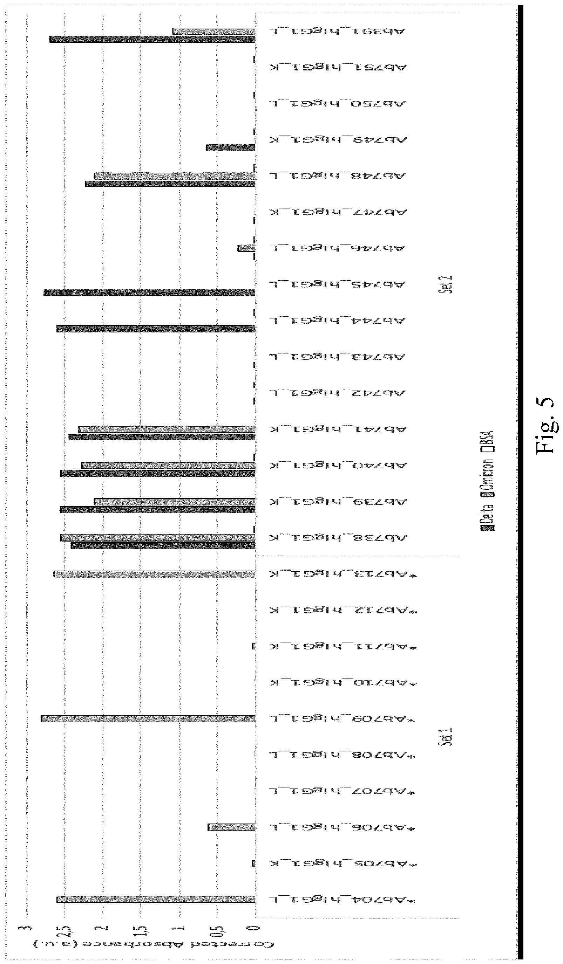

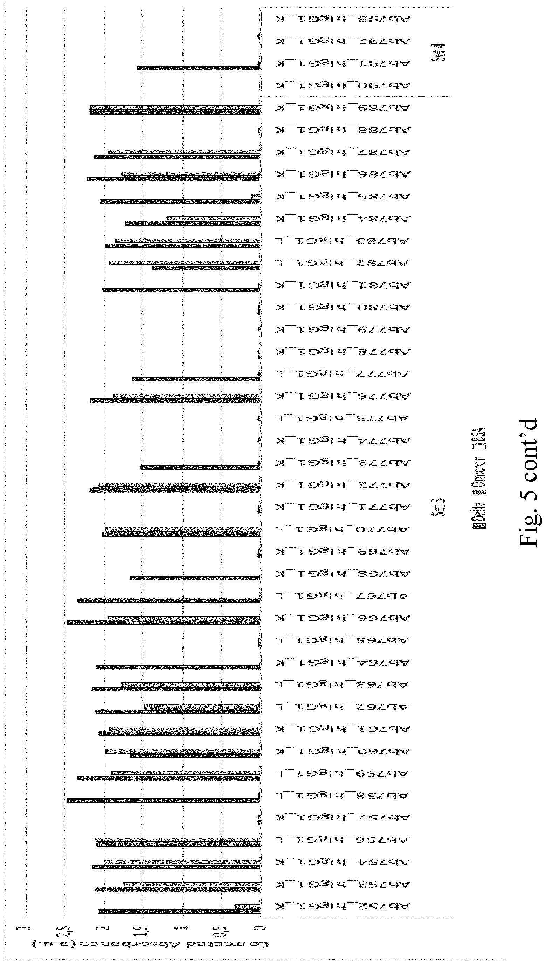

The antibody may comprise the heavy chain variable region (HCVR) and/or the light chain variable region (LCVR) of any one of the antibodies provided in Table 3. The antibody may be one shown in Figure 5 to have high bining to Delta spik protein, or more typically, high binding to Omicron spike protein, or still more typically, high binding to both Omicron and Delta spike protein, for example a corrected absorbance of at least 1.5, or at least 2, or at least 2 a.u. as shown in Figure 5. The antibody may be one shown in Table 2 to be in the same bin as antibody Ab390_ScFv_VL1, or in the same bin as antibody Ab371_hIgG1_L, or, most typically, the same bin as antibody Ab391_hIgG1_L.

The CDRs of the heavy chain (CDRH) and light chain variable domain (CDRL) are located at residues 27-38 (CDR1), residues 56-65 (CDR2) and residues 105-117 (CDR3) of each chain according to the IMGT numbering system (http://www.imgt.org; Lefranc MP, 1997, J, Immunol. Today, 18, 509). This numbering system is used in the present specification except where otherwise indicated.

The antibody of the invention may comprise at least four, five, or all six CDRs of an antibody selected from any one of Tables 3 or any of the other groups of antibodies described herein. The antibody may comprise at least one, at least two, or all three heavy chain CDRs (CDRHs). The antibody may comprise at least one, at least two, or all three light chain CDRs (CDRLs). The antibody typically comprises all six (i.e. three heavy and three light chain) CDRs.

The antibody of the invention may comprise a heavy chain variable region having ≥80%, ≥85%, ≥90%, ≥95%, ≥96%, ≥97%, ≥98%, ≥99% or 100% sequence identity to the heavy chain variable region amino acid sequence of any of these antibodies.

The antibody of the invention may comprise a light chain variable region having ≥80%, ≥85%, ≥90%, ≥95%, ≥96%, ≥97%, ≥98%, ≥99% or 100% sequence identity to the light chain variable region amino acid sequence of any of these antibodies.

For example, in some embodiments, the antibody comprises (a) a heavy chain variable region having at least 80% amino acid sequence identity to any one of SEQ ID NOs: 185, 321, 217, 249, 233, 81, 169, 353, 409, 457, 433, 137, 241, 97, 305, and 177, or comprising the amino acid sequence of any one of SEQ ID NOs: 185, 321, 217, 249, 233, 81, 169, 353, 409, 457, 433, 137, 241, 97, 305, and 177; and/or (b) a light chain variable region having at least 80% amino acid sequence identity to any one of SEQ ID NOs: 189, 325, 221, 253, 237, 85, 173, 357, 413, 461, 437, 141, 245, 101, 309, and 181 or comprising the amino acid sequence of any one of SEQ ID NOs: 189, 325, 221, 253, 237, 85, 173, 357, 413, 461, 437, 141, 245, 101, 309, and 181.

The antibody of the invention may comprise a heavy chain variable region having ≥80%, ≥85%, ≥90%, ≥95%, ≥96%, ≥97%, ≥98%, ≥99% or 100% sequence identity to the heavy chain variable region amino acid sequence of any of these antibodies and a light chain variable region having ≥80%, ≥85%, ≥90%, ≥95%, ≥96%, ≥97%, ≥98%, ≥99% or 100% sequence identity (or the same minimal percentage identity as defines the heavy chain variable region) to the matching light chain variable region amino acid sequence of the same antibody, as shown in Table 3. For example, in one embodiment, the antibody comprises heavy and light chain variable regions having the amino acid sequences of (a) SEQ ID NOs: 185 and 189; (b) SEQ ID NOs: 321 and 325; (c) SEQ ID NOs: 217 and 221; (d) SEQ ID NOs: 249 and 253; (e) SEQ ID NOs: 233 and 237; (f) SEQ ID NOs: 81 and 85; (g) SEQ ID NOs: 169 and 173; (h) SEQ ID NOs: 353 and 357; (i) SEQ ID NOs: 409 and 413; (j) SEQ ID NOs: 457 and 461; (k) SEQ ID NOs: 433 and 437; (1) SEQ ID NOs: 137 and 141; (m) SEQ ID NOs: 241 and 245; (n) SEQ ID NOs: 97 and 101; (o) SEQ ID NOs: 305 and 309; (p) SEQ ID NOs: 177 and 181.

Typically, the non-identical amino acids, as described above, of a variable region are not in the CDRs. Hence, an antibody of the invention may comprise 100% sequence identity over all six CDRs of a reference antibody from Table 3, but only ≥80%, ≥85%, ≥90%, ≥95%, ≥96%, ≥97%, ≥98%, ≥99% or 100% sequence identity over the complete length of the heavy and/or light chain variable regions of the same reference antibody.

An antibody of the invention may comprise a modification from the amino acid sequence of an antibody in Table 3, or any of the other groups of antibodies described herein, whilst maintaining the activity and/or function of the antibody. The modification may be a substitution, deletion and/or addition. For example, the modification may comprise 1, 2, 3, 4, 5, up to 10, up to 20, up to 30 or more amino acid substitutions and/or deletions from the amino acid sequence of the antibody in one of Tables 1 to 6. For example, the modification may comprise an amino acid substituted with an alternative amino acid having similar properties. Some properties of the 20 main amino acids, which can be used to select suitable substituents, are as follows:

| Ala | aliphatic, hydrophobic, neutral | Met | hydrophobic, neutral |
|---|---|---|---|
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged (+) | Trp | aromatic, hydrophobic, neutral |
| Leu | aliphatic, hydrophobic, neutral | Tyr | aromatic, polar, hydrophobic |

The modification may comprise a derivatised amino acid, e.g. a labelled or non-natural amino acid, providing the function of the antibody is not significantly adversely affected.

Modification of antibodies of the invention as described above may be prepared during synthesis of the antibody or by post-production modification, or when the antibody is in recombinant form using the known techniques of site-directed mutagenesis, random mutagenesis, or enzymatic cleavage and/or ligation of nucleic acids.

Antibodies of the invention may be modified (e.g. as described above) to improve the potency of said antibodies or to adapt said antibodies to new SARS-CoV-2 variants. The modifications may be amino acid substitutions to adapt the antibody to substitutions in a virus variant. For example, the known mode of binding of an antibody to the spike protein (e.g. by crystal structure determination, or modelling) may be used to identify the amino acids of the antibody that interact with the substitution in the virus variant. This information can then be used to identify possible substitutions of the antibody that will compensate for the change in the epitope characteristics. For example, a substitution of a hydrophobic amino acid in the spike protein to a negatively changes amino acid may be compensated by substituting the amino acid from the antibody that interacts with said amino acid in the spike protein to a positively charged amino acid. Methods for identifying residues of an antibody that may be substituted are known in the art.

Antibodies of the invention may be isolated antibodies. An isolated antibody is an antibody which is substantially free of other antibodies having different antigenic specificities.

The term 'antibody' as used herein may relate to whole, full-length antibodies (i.e. comprising two antibody heavy chains and two light chains inter-connected by disulphide bonds), as well as antigen-binding fragments thereof. The term may also encompass VHH antibodies (heavy-chain antibodies), having two heavy chains only and no light chains, and antigen binding fragments thereof. Antibodies typically comprise immunologically active portions of immunoglobulin (Ig) molecules, i.e., molecules that contain an antigen binding site that specifically binds (immunoreacts with) an antigen. By "specifically binds" or "immunoreacts with" is meant that the antibody reacts with one or more antigenic determinants of an antigen, i.e. in the normal way that antibodies bind to antigen. Each heavy chain is typically comprised of a heavy chain variable region (abbreviated herein as HCVR or VH) and at least one heavy chain constant region. Each light chain is typically comprised of a light chain variable region (abbreviated herein as LCVR or VL) and a light chain constant region. Typically the variable regions of the heavy and light chains contain a binding domain that interacts with an antigen, except for VHH antibodies where the variable regions of the heavy chains only comprise the antigen binding domain. The VH and VL regions can be further subdivided into regions of hypervariability, termed complementarity determining regions (CDR), interspersed with regions that are more conserved, termed framework regions (FR). Antibodies may include, but are not limited to, polyclonal, monoclonal, chimeric, recombinant, dAb (domain antibody), single chain, Fab (fragment antigen-binding regions), Fab' and F(ab')2 fragments, scFvs (single chain variable fragments), and Fab expression libraries.

An antibody of the invention may be a monoclonal antibody. Monoclonal antibodies (mAbs) of the invention may be produced by a variety of techniques, including conventional monoclonal antibody methodology, for example those disclosed in "Monoclonal Antibodies: a manual of techniques"(Zola H, 1987, CRC Press) and in "Monoclonal Hybridoma Antibodies: techniques and applications" (Hurrell JGR, 1982 CRC Press).

An antibody of the invention may be a multivalent antibody. "Multivalent antibody," as used herein, is an antibody comprising three or more antigen binding sites. The multivalent antibody may be engineered to have the three or more antigen binding sites. The antibodies of the present invention can be multivalent antibodies (which are other than of the IgM class) with three or more antigen binding sites (e.g. "trivalent", "trimeric", "tetravalent" or "tetrameric" antibodies). Such antibodies can be produced by recombinant expression of nucleic acid encoding the polypeptide chains of the antibody, or using methods described further below and known in the art. The multivalent antibody can comprise a dimerization domain and three or more antigen binding sites. The dimerization domain may comprise (or consists of) an Fc region or a hinge region. The antibody will then comprise an Fc region and three or more antigen binding sites amino-terminal to the Fc region. The multivalent antibody may comprise or consist of three to about eight antigen binding sites. The multivalent antibody comprises at least one polypeptide chain or may comprise two polypeptide chains, wherein the polypeptide chain(s) comprise two or more variable domains. For instance, the polypeptide chain(s) may comprise VD1-(X1)n-VD2-(X2)n-Fc, wherein VD1 is a first variable domain, VD2 is a second variable domain, Fc is one polypeptide chain of an Fc region, XI and X2 represent an amino acid or polypeptide, and n is 0 or 1. For instance, the polypeptide chain(s) may comprise: VH-CH1-flexible linker-VH-CHI-Fc region chain; or VH-CH 1-VH-CH 1-Fc region chain. Or VH-CH1-CH2-CH3(Fc region)-Flexible linker1-VH-Flexible linker2-VL.The multivalent antibody may further comprises at least two, or four light chain variable domain polypeptides. The multivalent antibody may, for instance, comprise from about two to about eight light chain variable domain polypeptides. The light chain variable domain polypeptides may comprise a light chain variable domain and, optionally, further comprise a CL domain. The multivalent antibody may be IgG.

In one particular embodiment, each antigen binding site of the the multivalent antibody comprises one of the following sets of CDRs: (a) SEQ ID NOs: 186, 187, 188, 190, 191 and 192; (b) SEQ ID NOs: 322, 323, 324, 326, 327 and 328; (c) SEQ ID NOs: 218, 219, 220, 222, 223 and 224; (d) SEQ ID NOs: 250, 251, 252, 254, 255 and 256; (e) SEQ ID NOs: 234, 235, 236, 238, 239 and 240; and (f) SEQ ID NOs: 82, 83, 84, 86, 87 and 88.

An antibody or multivalent antibody of the invention may be a multispecific antibody, for example, a bispecific antibody or a trispecific antibody. "Multispecific antibody" is an antibody having at least two different binding sites, each site with a different binding specificity. A multispecific antibody can be a full-length antibody or an antibody fragment, and the different binding sites may bind each to a different antigen or the different binding sites may bind to two different epitopes of the same antigen. In some cases a bispecific antibody of the invention may bind to two different antigens. For example, one 'arm' of the body binds the spike protein of SARS-CoV-2, and the other 'arm' binds a different antigen. In one embodiment, a bispecific antibody of the invention may bind to two separate (non-overlapping) epitopes on the spike protein. In one embodiment, a bispecific antibody of the invention binds to the NTD of the spike protein with one 'arm' and to the RBD of the spike protein with another 'arm'. In one embodiment, a bispecific antibody of the invention binds to two different epitopes on the RBD of the spike protein. In one embodiment, a bispecific antibody of the invention binds to different proteins with each 'arm'. One or more (e.g. two) antibodies/fragments of the invention can be coupled to form a multispecific (e.g. bispecific) antibody.

In another embodiment, the antibody or fragment comprises (i) one antigen-binding site comprising a set of CDRH1, CDRH2, CDRH3, CDRL1, CDRL2 and CDRL3 amino acid sequences selected from (I) SEQ ID NOs: 186, 187, 188, 190, 191 and 192, (II) SEQ ID NOs: 322, 323, 324, 326, 327 and 328, (III) SEQ ID NOs: 218, 219, 220, 222, 223 and 224, and (IV) SEQ ID NOs: 250, 251, 252, 254, 255 and 256; and (ii) one antigen-binding site comprising the set of CDRH1, CDRH2, CDRH3, CDRL1, CDRL2 and CDRL3 amino acid sequences of SEQ ID NOs: 82, 83, 84, 86, 87 and 88. Hence, the antibody may be a bispecific antibody. The antibody may comprise (i) heavy and light chain variable regions having a pair of sequences selected from: (I) SEQ ID NOs: 185 and 189 sequences of SEQ ID NOs: 185 and 189; (II) SEQ ID NOs: 321 and 325; (III) SEQ ID NOs: 217 and 221; (IV) SEQ ID NOs: 249 and 253; and (ii) heavy and light chain variable regions having the sequences of SEQ ID NOs: 81 and 85.

In another embodiment, the antibody or fragment comprises (i) one antigen-binding site comprising a set of CDRH1, CDRH2, CDRH3, CDRL1, CDRL2 and CDRL3 amino acid sequences selected from (I) SEQ ID NOs: 186, 187, 188, 190, 191 and 192, (II) SEQ ID NOs: 322, 323, 324, 326, 327 and 328, (III) SEQ ID NOs: 218, 219, 220, 222, 223 and 224, and (IV) SEQ ID NOs: 250, 251, 252, 254, 255 and 256; (ii) one antigen-binding site comprising the set of CDRH1, CDRH2, CDRH3, CDRL1, CDRL2 and CDRL3 amino acid sequences of SEQ ID NOs: 234, 235, 236, 238, 239 and 240. Hence, the antibody may be a trispecific antibody. The antibody may comprise (i) heavy and light chain variable regions having a pair of sequences selected from: (I) SEQ ID NOs: 185 and 189 sequences of SEQ ID NOs:

185 and 189; (II) SEQ ID NOs: 321 and 325; (III) SEQ ID NOs: 217 and 221; (IV) SEQ ID NOs: 249 and 253; and (ii) heavy and light chain variable regions having the sequences of SEQ ID NOs: 233 and 237.

In another embodiment, the antibody or fragment comprises (i) one antigen-binding site comprising the set of CDRH1, CDRH2, CDRH3, CDRL1, CDRL2 and CDRL3 amino acid sequences of SEQ ID NOs: 82, 83, 84, 86, 87 and 88; and (ii) one antigen-binding site comprising the set of CDRH1, CDRH2, CDRH3, CDRL1, CDRL2 and CDRL3 amino acid sequences of SEQ ID NOs: 234, 235, 236, 238, 239 and 240. Hence, the antibody may be a bispecific antibody. The antibody may comprise (i) heavy and light chain variable regions having the sequences of SEQ ID NOs 81 and 85; and (ii) heavy and light chain variable regions having the sequences of SEQ ID NOs: 233 and 237.

An antibody of the invention may be a trispecific antibody. In one embodiment, a trispecific antibody of the invention may bind to three separate (non-overlapping) epitopes on the spike protein. In one embodiment, a trispecific antibody of the invention binds to three different epitopes on the RBD of the spike protein. For example, three antibodies/fragments of the invention can be coupled to form a trispecific antibody.

In one embodiment, the antibody or fragment comprises (i) one antigen-binding site comprising a set of CDRH1, CDRH2, CDRH3, CDRL1, CDRL2 and CDRL3 amino acid sequences selected from (I) SEQ ID NOs: 186, 187, 188, 190, 191 and 192, (II) SEQ ID NOs: 322, 323, 324, 326, 327 and 328, (III) SEQ ID NOs: 218, 219, 220, 222, 223 and 224, and (IV) SEQ ID NOs: 250, 251, 252, 254, 255 and 256; (ii) one antigen-binding site comprising the set of CDRH1, CDRH2, CDRH3, CDRL1, CDRL2 and CDRL3 amino acid sequences of SEQ ID NOs: 82, 83, 84, 86, 87 and 88; and (iii) one antigen-binding site comprising the set of CDRH1, CDRH2, CDRH3, CDRL1, CDRL2 and CDRL3 amino acid sequences of SEQ ID NOs: 234, 235, 236, 238, 239 and 240. Hence, the antibody may be a trispecific antibody. The antibody may comprise (i) heavy and light chain variable regions having a pair of sequences selected from: (I) SEQ ID NOs: 185 and 189 sequences of SEQ ID NOs: 185 and 189; (II) SEQ ID NOs: 321 and 325; (III) SEQ ID NOs: 217 and 221; (IV) SEQ ID NOs: 249 and 253; (ii) heavy and light chain variable regions having the sequences of SEQ ID NOs: 81 and 85; and (iii) heavy and light chain variable regions having the sequences of SEQ ID NOs: 233 and 237.

Techniques for making multispecific and multivalent antibodies include, but are not limited to, recombinant co-expression of two immunoglobulin heavy chain-light chain pairs having different specificities (see e.g., Milstein and Cuello, Nature 305: 537 (1983), WO 93/08829, and Traunecker et al., EMBOJ. 10: 3655 (1991)). "Knob-in-hole" engineering can also be used (see, e.g., U.S. Patent No. 5,731,168). In this method, "knobs" are constructed by replacing small amino acid side chains from the interface of a first polynucleotide with larger side chains (e.g. tyrosine or tryptophan). Complementary "holes" of identical or similar size to the "knobs" are optionally created on the interface of the second polynucleotide by replacing large amino acid chains with smaller ones (e.g. alanine or threonine). Where a suitably positioned and dimensioned "knob" or "hole" exists at the interface of either the first or the second polynucleotide, it is only necessary to engineer a corresponding "hole" or "knob", respectively, at the adjacent interface. Accordingly, a multispecific or multivalent antibody of the invention may comprise a first polynucleotide and a second polynucleotide which meet at an interface, wherein the first polynucleotide has a "knob" at the interface thereof which is positionable in a "hole" at the interface of the second polynucleotide.

Multispecific antibodies can also be made by engineering "electrostatic steering" effects that favor formation of Fc-heterodimeric antibody molecules rather than homodimers (WO 2009/089004A1); cross-linking two or more antibodies or fragments (see, e.g., US Pat. No. 4,676,980, and Brennan et al., Science, 229: 81 (1985)); using leucine zippers to produce bispecific antibodies (see, e.g., Kostelny et al., J. Immunol, 148(5): 1547-1553 (1992)); using "diabody" technology for making bispecific antibody fragments (see, e.g., Hollinger et al., Proc. Natl. Acad. Sci. USA, 90:6444-6448 (1993)); using single-chain Fv (scFv) dimers (see, e.g. Gruber et al., J. Immunol, 152:5368 (1994)); or tri-specific antibodies (see e.g., Tutt et al., J. Immunol. 147: 60 (1991).

Multispecific antibodies of the invention can also be made by fusing a classical antibody to the C-terminus of an ScFv antibody.

It is contemplated that any of the anti-SARS-CoV-2 antibodies of the present invention can be prepared as multispecific antibodies using the methods and techniques known in the art and/or described herein.

A multispecific antibody of the present invention may comprise an hIgG Fc region. The hIgG region may be a LaLaNa region or variant thereof, for example as used in SEQ ID NO: 1529. The constant region domains of an antibody molecule of the invention, if present, may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses where antibody effector functions are required. Alternatively, IgG2 and IgG4 isotypes may be used when the antibody molecule is intended for therapeutic purposes and antibody effector functions are not required. Typically, the constant regions are of human origin. In particular, human IgG (i.e. IgG1, IgG2, IgG3 or IgG4) constant region domains may be used. Most typically, a human IgG1 constant region is used, or mutant variant thereof that lacks effector functions, such as a variant having the VH domain LaLaNa mutation of the human Fc of IgG1.

The light chain constant region may be either lambda or kappa. The inventors found a strong bias for the use of Lamda light chains in the antibodies described in PCT/EP2022/062777. Hence the light chain constant region may typically be lambda.

An antibody of the invention may be a chimeric antibody, a CDR-grafted antibody, a nanobody, a human or humanised antibody. Typically, the antibody is a human antibody. Fully human antibodies are those antibodies in which the variable regions and the constant regions (where present) of both the heavy and the light chains are all of human origin, or substantially identical to sequences of human origin, but not necessarily from the same antibody.

The antibody of the invention may be an antigen-binding fragment. An antigen-binding fragment of the invention binds to the same epitope as a reference full length antibody or parent antibody, i.e. the antibody from which the antigen-binding fragment is derived. An antigen-binding fragment of the invention typically retains the parts of the parent antibody that interact with the epitope. Typically, the antigen-binding fragment retains the same or similar binding affinity to the antigen as the reference antibody. Methods for creating and manufacturing antibody fragments are well known in the art (see for example Verma R et al., 1998, J. Immunol. Methods, 216, 165-181).

An antigen-binding fragment does not necessarily have an identical sequence to the parent antibody. In one embodiment, the antigen-binding fragment may have ≥70%, ≥80%, ≥85%, ≥90%, ≥95%, ≥96%, ≥97%, ≥98%, ≥99%, 100% sequence identity with the respective variable region domains of the parent antibody. Typically, the non-identical amino acids of a variable region are not in the CDRs.

Methods for screening antibodies of the invention that do not share 100% amino acid sequence identity with one of the antibodies disclosed herein, that possess the desired specificity, affinity and functional activity include enzyme linked immunosorbent assays, biacore, focus reduction neutralisation assay (FRNT), and other techniques known within the art or described herein.

An antibody of the invention is typically able to neutralise at least one biological activity of SAR-CoV-2 (a neutralising antibody), particularly to neutralise virus infectivity or block binding of the spike protein or a binding portion thereof, e.g. S1 or RBD, to the ACE2-receptor or a RBD-binding portion thereof. Blocking of the interaction between spike and ACE2-receptor can be total or partial. For example, an antibody of the invention may reduce spike-ACE2 formation by ≥30%, ≥40%, ≥50%, ≥60%, ≥70%, ≥80%, ≥90%, ≥95%, ≥99% or 100%. Blocking of spike-ACE2 formation can be determined or measured by any suitable means known in the art, or described herein, e.g. using an ELISA binding or neutralisation assay, for example as described in the Examples herein.

An antibody of the invention may have an affinity constant ($K_D$) value for the spike protein of SARS-CoV-2 of ≤5nM, ≤4nM, ≤3nM, ≤2nM, ≤1nM, ≤0.5nM, <0.4nM, <0.3nM, <0.2nM, <0.1nM or <0.05nM.

The KD value can be measured by any suitable means known in the art, for example, by ELISA or Surface Plasmon Resonance (Biacore) at 25 °C.

Antibodies of the invention may have any combination of one or more of the properties described herein.

Antibodies of the invention may bind to the same epitope as, or compete for binding to SARS-CoV-2 spike protein with, any one of the antibodies described herein (i.e. in particular with antibodies having the CDR sequences or the heavy and light chain variable regions described above).

The skilled person is able to determine the binding site (epitope) of an antibody, whether an antibody binds to the same epitope as, or competes for binding with, an antibody described herein, using techniques known in the art.

For example, to determine if a test antibody binds to the same epitope as an antibody described herein (referred to a "reference antibody" in the following paragraphs), the reference antibody may be allowed to bind to a protein or peptide under saturating conditions. Next, the ability of a test antibody to bind to the protein or peptide is assessed. If the test antibody is able to bind to the protein or peptide following saturation binding with the reference antibody, it can be concluded that the test antibody binds to a different epitope than the reference antibody. On the other hand, if the test antibody is not able to bind to protein or peptide following saturation binding with the reference antibody, then the test antibody may bind to the same epitope as the epitope bound by the reference antibody of the invention.

To determine if an antibody competes for binding with a reference antibody, the above-described binding methodology is performed in two orientations. In a first orientation, the reference antibody is allowed to bind to a protein/peptide under saturating conditions followed by assessment of binding of the test antibody to the protein/peptide molecule. In a second orientation, the test antibody is allowed to bind to the protein/peptide under saturating conditions followed by assessment of binding of the reference antibody to the protein/peptide. If, in both orientations, only the first (saturating) antibody is capable of binding to the protein/peptide, then it is concluded that the test antibody and the reference antibody compete for binding to the protein/peptide. As will be appreciated by the skilled person, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

As well as sequences defined by percentage identity or number of sequence changes, the invention further provides an antibody defined by its ability to cross-compete with one of the specific antibodies set out herein. It may be that the antibody also has one of the recited levels of sequence identity or number of sequence changes as well.

Cross-competing antibodies can be identified using any suitable method in the art, for example by using competition ELISA or BIAcore assays where binding of the cross competing antibody to a particular epitope on the spike protein prevents the binding of an antibody of the invention or *vice versa.* In one embodiment, the antibody produces ≥50%, ≥60%, ≥70%, ≥80%, ≥90% or 100% reduction of binding of the specific antibody disclosed herein.

Appropriate aantibodies described below in the Examples may be used as reference antibodies.

Other techniques that may be used to determine antibody epitopes include hydrogen/deuterium exchange, X-ray crystallography and peptide display libraries. A combination of these techniques may be used to determine the epitope of the test antibody.

The approaches described herein could be applied equally to other data, e.g. surface plasmon resonance or ELISA, and provides a general way of rapidly determining locations from highly redundant competition experiments.

Combinations of Antibodies

In some cases, the invention relates to a combination of any two or more, e.g. any 2, 3, 4, 5, 6, 7, or 8 of the antibodies of the invention described herein. The combination antibodies may be selected from any of those provided in Table 3, or have relevant amino acid sequences thereof as described herein, e.g. the six CDR sequences or the heavy and/or light chain variable sequences of these antibodies, and/or sequences sharing high sequence identity with the heavy and/or light chain variable sequences of these antibodies, as described herein.

In some cases the combination of antibodies comprises or consists of two or more antibodies selected from those designated herein as Ab754, Ab759, Ab763, Ab761 and Ab772; or selected from Ab754, Ab759, Ab763, Ab761, Ab772 and Ab738; or selected from Ab752, Ab776, Ab789, Ab783, Ab786, Ab762, Ab748 and Ab740; or selected from Ab754, Ab759, Ab763, Ab761, Ab772, Ab752, Ab776, Ab789, Ab783, Ab786, Ab762, Ab748 and Ab740; or selected from Ab759, Ab763, Ab761, Ab772, Ab738Ab752, Ab776, Ab789, Ab783, Ab786, Ab762, Ab748 and Ab740; or selected from Ab738Ab752, Ab776, Ab789, Ab783, Ab786, Ab762, Ab748 and Ab740; or selected from Ab770 and Ab753; or selected from Ab754, Ab759, Ab763, Ab761, Ab772 Ab770 and Ab753; or selected from Ab754, Ab759, Ab763, Ab761, Ab772, Ab738, Ab770 and Ab753; or selected from Ab754, Ab759, Ab763, Ab761, Ab772, Ab752, Ab776, Ab789, Ab783, Ab786, Ab762, Ab748, Ab740, Ab770 and Ab753; or selected from Ab754, Ab759, Ab763, Ab761, Ab772, Ab738, Ab752, Ab776, Ab789, Ab783, Ab786, Ab762, Ab748, Ab740, Ab770 and Ab753; or selected from Ab738, Ab770 and Ab753; or selected from Ab752, Ab776, Ab789, Ab783, Ab786, Ab762, Ab748, Ab740, Ab770 and Ab753; or selected from Ab738, Ab752, Ab776, Ab789, Ab783, Ab786, Ab762, Ab748, Ab740, Ab770 and Ab753, or any other combination of antibodies as described herein; or combinations of antibodies having the relevant sequences of these sets of antibodies, as described herein.

In some cases the combination of antibodies includes antibodies from any combination of two of the three bins shown in Table 2, or comprises or consists of one antibody from each of the three bins. In one embodiment the combination of antibodies comprises one or more antibodies selected from any two or more of the following groups (i) to (iii):

(i) antibodies having the following sets of six CDRs sequences: (a) SEQ ID NOs: 186, 187, 188, 190, 191 and 192; (b) SEQ ID NOs: 322, 323, 324, 326, 327 and 328; (c) SEQ ID NOs: 218, 219, 220, 222, 223 and 224; (d) SEQ ID NOs: 250, 251, 252, 254, 255 and 256; (g) SEQ ID NOs: 170, 171, 172, 174, 175 and 176; (h) SEQ ID NOs: 354, 355, 356, 358, 359 and 360; (i) SEQ ID NOs: 410, 411, 412, 414, 415 and 416; (j) SEQ ID NOs: 458, 459, 460, 462, 463 and 464; and (k) SEQ ID NOs: 434, 435, 436, 438, 439 and 440; or the following heavy and light chain variable sequences: (a) SEQ ID NOs: 185 and 189; (b) SEQ ID NOs: 321 and 325; (c) SEQ ID NOs: 217 and 221; (d) SEQ ID NOs: 249 and 253; (g) SEQ ID NOs: 169, 171, and 173; (h) SEQ ID NOs: 353 and 357; (i) SEQ ID NOs: 409 and 413; (j) SEQ ID NOs: 457 and 461; and (k) SEQ ID NOs: 433 and 437; or antibodies having heavy and light variable chain sequences having at least at least 70%, at least 80%, at least 85%, at least 90%, at least 95%, at least 98%, or at least 99% sequence identity therewith;

(ii) antibodies having the following sets of six CDRs sequences: (f) SEQ ID NOs: 82, 83, 84, 86, 87 and 88; (m) SEQ ID NOs: 242, 243, 244, 246, 247 and 248; and (n) SEQ ID NOs: 98, 99, 100, 102, 103 and 104; or the following heavy and light chain variable sequences: (f) SEQ ID NOs: 81 and 85; (m) SEQ ID NOs: 241 and 245; and (n) SEQ ID NOs: 97 and 101; and

(iii) antibodies having the following sets of six CDRs sequences: (e) SEQ ID NOs: 234, 235, 236, 238, 239 and 240; (1) SEQ ID NOs: 138, 139, 140, 142, 143 and 144; (o) SEQ ID NOs: 306, 307, 308, 310, 311 and 312; and (p) SEQ ID NOs: 178, 179, 180, 182, 183 and 184; or the following heavy and light chain variable sequences: (e) SEQ ID NOs: 233 and 237; (1) SEQ ID NOs: 137 and 141; (o) SEQ ID NOs: 305 and 319; and (p) SEQ ID NOs: 177 and 181.

In one embodiment the combination of antibodies comprises one or more antibodies selected from any two or more of the following groups (i) to (iii):

(i) antibodies having the following sets of six CDRs sequences: (a) SEQ ID NOs: 186, 187, 188, 190, 191 and 192; (b) SEQ ID NOs: 322, 323, 324, 326, 327 and 328; (c) SEQ ID NOs: 218, 219, 220, 222, 223 and 224; (d) SEQ ID NOs: 250, 251, 252, 254, 255 and 256;; or the following heavy and light chain variable sequences: (a) SEQ ID NOs: 185 and 189; (b) SEQ ID NOs: 321 and 325; (c) SEQ ID NOs: 217 and 221; (d) SEQ ID NOs: 249 and 253; or antibodies having heavy and light variable chain sequences having at least at least 70%, at least 80%, at least 85%, at least 90%, at least 95%, at least 98%, or at least 99% sequence identity therewith;
(ii) antibodies having the following sets of six CDRs sequences: (f) SEQ ID NOs: 82, 83, 84, 86, 87 and 88; or the following heavy and light chain variable sequences: (f) SEQ ID NOs: 81 and 85; or antibodies having heavy and light variable chain sequences having at least at least 70%, at least 80%, at least 85%, at least 90%, at least 95%, at least 98%, or at least 99% sequence identity therewith; and
(iii) antibodies having the following sets of six CDRs sequences: (e) SEQ ID NOs: 234, 235, 236, 238, 239 and 240; or the following heavy and light chain variable sequences: (e) SEQ ID NOs: 233 and 237; or antibodies having heavy and light variable chain sequences having at least at least 70%, at least 80%, at least 85%, at least 90%, at least 95%, at least 98%, or at least 99% sequence identity therewith.

In one embodiment, the combination of antibodies comprises or consists of those having one or more other functional characteristics described herein.

A combination of the antibodies of the invention may be useful as a therapeutic cocktail. Hence, the invention also provides a pharmaceutical composition comprising a combination of the antibodies of the invention, as explained further below.

A combination of the antibodies of the invention may be useful for diagnosis. Hence, the invention also provides a diagnostic kit comprising a combination of the antibodies of the invention. Also provided herein are methods of diagnosing a disease or complication associated with coronavirus infections in a subject, as explained further below.

SARS-CoV-2 Variants

Where not otherwise specified herein, the SARS-CoV-2 strain referred to herein is SARS-CoV-2 (2019-nCoV), Wuhan-Hu-1 strain, having the spike protein amino acid sequence of SEQ ID NO: 2081 and variants described herein are defined relative to this strain or to any other strain having the same spike protein sequence as SARS-CoV-2 (2019-nCoV) (SEQ ID NO: 2081).

Polynucleotides, vectors and host cells

The invention also provides one or more isolated polynucleotides (e.g. DNA or RNA) encoding an antibody of the invention. In some cases, the complete sequence encoding the complete antibody may be spread across more than one polynucleotide, but the polynucleotides combined are able to encode an antibody of the invention. For example, the polynucleotides may encode the heavy and/or light chain variable regions(s) of an antibody of the invention. The polynucleotides may encode the full heavy and/or light chain of an antibody of the invention. Typically, one polynucleotide would encode each of the heavy and light chains.

In particular embodiments, the polynucleotide(s) of the invention comprise or consist of the polynucleotide sequences of any one of SEQ ID NOs: 2082 to 2094. The skilled person is readily able to use these sequences and the amino acid sequences of other antibodies disclosed herein, to arrive at suitable polynucleotides encoding other antibodies described herein. Other suitable vector sequences that may be used are provided in WO 2022/238481.

Polynucleotides which encode an antibody of the invention can be obtained by methods well known to those skilled in the art. For example, DNA sequences coding for part or all of the antibody heavy and light chains may be synthesised as desired from the corresponding amino acid sequences.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

A polynucleotide of the invention may be provided in the form of an expression cassette, which includes control sequences operably linked to the inserted sequence, thus allowing for expression of the antibody of the invention *in vivo*. Hence, the invention also provides one or more expression cassettes encoding the one or more polynucleotides that encode an antibody of the invention. These expression cassettes, in turn, are typically provided within vectors (e.g. plasmids or recombinant viral vectors). Hence, in one embodiment, the invention provides a vector encoding an antibody of the invention. In another embodiment, the invention provides vectors which collectively encode an antibody of the invention. The vectors may be cloning vectors or expression vectors. A suitable vector may be any vector which is capable of carrying a sufficient amount of genetic information, and allowing expression of a polypeptide of the invention.

The polynucleotides, expression cassettes or vectors of the invention are introduced into a host cell, e.g. by transfection. Hence, the invention also provides a host cell comprising the one or more polynucleotides, expression cassettes or vectors of the invention. The polynucleotides, expression cassettes or vectors of the invention may be introduced transiently or permanently into the host cell, allowing expression of an antibody from the one or more polynucleotides, expression cassettes or vectors. Such host cells include transient, or preferably stable higher eukaryotic cell lines, such as mammalian cells or insect cells, lower eukaryotic cells, such as yeast, or prokaryotic cells, such as bacteria cells. Particular examples of cells include mammalian HEK293, such as HEK293F, HEK293T, HEK293S or HEK Expi293F, CHO, HeLa, NS0 and COS cells, or any other cell line used herein, such as the ones used in the Examples. Preferably the cell line selected will be one which is not only stable, but also allows for mature glycosylation.

The invention also provides a process for the production of an antibody of the invention, comprising culturing a host cell containing one or more vectors of the invention under conditions suitable for the expression of the antibody from the one or more polynucleotides of the invention, and isolating the antibody from said culture.

Pharmaceutical composition

The invention provides a pharmaceutical composition comprising an antibody of the invention or a combination of the antibodies of the invention. The pharmaceutical compositions or kits described herein may comprise, in addition to one or more antibodies, a pharmaceutically acceptable excipient, carrier, diluent, buffer, stabiliser, preservative, adjuvant or other materials well known to those skilled in the art. Such materials are preferably non-toxic and preferably do not interfere with the pharmaceutical activity of the active ingredient(s). Examples of suitable aqueous carriers include water, buffered water and saline. The composition of the invention may include one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts. Other suitable pharmaceutically acceptable carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be desirable to include isotonic agents, for example, sugars, polyalcohols such as mannitol, sorbitol, or sodium chloride in the composition. The precise nature of the carrier or other material may depend on the route of administration.

Therapeutic compositions typically must be sterile and stable under the conditions of manufacture and storage. The composition can be formulated as a solution, microemulsion, liposome, or other ordered structure suitable to high drug concentration.

Pharmaceutical compositions of the invention may comprise additional therapeutic agents, for example an anti-viral agent. The anti-viral agent may bind to coronavirus and inhibit viral activity. Alternatively, the anti-viral agent may not bind directly to coronavirus but still affect viral activity/infectivity. The anti-viral agent could be a further anti-coronavirus antibody, which binds somewhere on SARS-CoV-2 other than the spike protein. Examples of an anti-viral agent useful with the invention include Remdesivir, Lopinavir, ritonavir, APN01, and Favilavir. In other cases the additional therapeutic agent may be an anti-inflammatory agent, such as a corticosteroid (e.g. Dexamethasone) or a non-steroidal anti-inflammatory drug (e.g. Tocilizumab), or the additional therapeutic agent may be an anti-coronavirus vaccine.

The pharmaceutical composition may be administered subcutaneously, intravenously, intradermally, intramuscularly, intranasally or orally.

Also within the scope of the invention are kits comprising antibodies or other compositions of the invention and instructions for use, for example in any method of the invention. The kit may further contain one or more additional reagents, such as an additional therapeutic or prophylactic agent as discussed herein.

Methods and uses of the invention

The invention further relates to the use of the antibodies or combinations of antibodies or pharmaceutical compositions described herein, e.g. in a method for treatment of the human or animal body by therapy, or in a diagnostic method. The term "treatment" as used herein includes therapeutic and prophylactic treatment. Administration is typically in a "prophylactically effective amount" or a "therapeutically effective amount" (although prophylaxis may be considered therapy/treatment), this being sufficient to result in a clinical response or to show clinical benefit to the individual, e.g.

an effective amount to prevent or delay an infection, or the onset of a disease or condition, to ameliorate one or more symptoms, to induce or prolong remission, or to delay relapse or recurrence. The methods and uses of the invention may comprise inhibiting the disease state (such as COVID-19), e.g. arresting its development; and/or relieving the disease state (such as COVID-19), *e.g.* causing regression of the disease state, e.g. until a desired endpoint is reached. The methods and uses of the invention may comprise the amelioration or the reduction of the severity, duration or frequency of a symptom of the disease state (such as COVID-19) (*e.g.* lessen the pain or discomfort), and such amelioration may or may not be directly affecting the disease. The symptoms or complications may be fever, headache, fatigue, loss of appetite, myalgia, diarrhoea, vomiting, abdominal pain, dehydration, respiratory tract infections, cytokine storm, acute respiratory distress syndrome (ARDS) sepsis, and/or organ failure (e.g. heart, kidneys, liver, GI, lungs). In some cases the methods and uses of the invention may lead to a decrease in the viral load of coronavirus (e.g. SARS-CoV-2), e.g. by ≥10%, ≥20%, ≥30%, ≥40%, ≥50%, ≥60%, ≥70%, ≥80%, ≥90%, or 100% compared to pre-treatment. Methods of determining viral load are well known in the art, e.g. infection assays. In some cases, the methods and uses of the invention may comprise preventing the coronavirus infection, or a disease or condition associated therewith, from occurring in a subject (e.g. humans), in particular, when such subject is predisposed to complications associated with coronavirus infection.

In some embodiments, the invention relates to methods of treating or preventing a coronavirus or beta-coronavirus (e.g. SARS-CoV-2) infection, or a disease or complication associated therewith, e.g. COVID-19. In one embodiment the invention relates to methods of treating or preventing infection by a virus that expresses a spike protein, an S1 domain, or an RBD having an epitope that is specifically bound by an antibody of the invention, or a disease or complication associated therewith. Any suitable SARS-CoV-2 strain may be treated, for example any strain described herein, such as in the Examples. Hence, in some cases the invention relates to methods of treating or preventing infection with SARS-CoV-2 (2019-nCoV), Wuhan-Hu-1 strain, or a mutant or variant thereof comprising any one or more of the following mutations in the reference spike protein amino acid sequence of SEQ ID NO: 2081: N501Y, K417N, E484K, and Y453F, or a strain comprising mutations N501Y, K417N and E484K. The RBD of the strain may have additional mutations, such as amino acid additions, substitutions or deletions. The RBD of the strain typically has at least 80%, or at least 85%, 90%, 92%, 95%, 96%, 97%, 98%, or 99% sequence of identity to SEQ ID NO: 2081, or to one of the specific variants of SEQ ID NO: 2081 described herein, e.g. having one of more of substitutions N501Y, K417N, E484K, and Y453F. Other mutations in the spike protein of known SARS-CoV-2 strains, relative to SEQ ID NO: 2081, that may be treated are: deletion of residues 69-70; deletions of residues 69-70 and substitution N501Y; deletion of residue 144; substitution E484K; substitution A570D; substitution D614G; substitution P681H; substitution T716I; substitution S982A; substitution D1118H; substitutions K417N, E484K, N501Y, L18F, D80G, D215G, R246I, D614G, and/or A701V and/or deletion of residue 242-244; deletion of residues 242-244 and substitution N501Y; deletion of residues 242-244 and substitution E484K; substitutions K417T, E484K, N501Y, L18F, T20N, P26S, D138Y, R190S, H655Y, and/or T1027I; substitutions L18F, T20N, P26S, D138Y, R190S, K417T, E484K, N501Y D614G, H655Y, T1027I, and/or V1176F; L425R and/or E484Q; Spike D614G, Spike E154K, Spike E484Q, Spike Spike L452R and Spike P681R; Spike D614G, Spike E154K, Spike E484Q, Spike E1072K, Spike K1073R, Spike L452R, Spike P681R; Spike D614G, Spike E154K, Spike E484Q, Spike G142D, Spike H1101D, Spike L452R, Spike P681R and Spike Q1071H; HV69-70 deletion, N501Y and D614G; H69del, V70del, Y144del, N501Y, A570D, D614G and P681H; H69del, V70del, Y144del, N501Y, A570D, D614G, P681H, T716I, S982A and D1118H; K417N, E484K and N501Y; K417N, E484K, N501Y and D614G; L18F, T20N, P26S, D138Y, R190S, K417T, E484K, N501Y, D614G, H655Y, T1027I and V1176F; ΔH69/ΔV70, Y453F and D614G; and substitution of lysine (K) at position 417 to asparagine (N) or threonine (T). In some cases the SARS-CoV-2 strain may be the Omirkron variant having the following mutations: A67V, HV69-70del, T95I, G142D, VYY143-145del, N211del, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F.

The SARS-CoV-2 strain may in some cases comprise any one or any combination of mutations described herein.

The method may comprise administering a therapeutically or prophylactically effective amount of an antibody, a combination of antibodies, or a pharmaceutical composition of the invention. In some cases, the method comprises identifying the presence of coronavirus in a sample, e.g. SARS-CoV-2, from the subject, for example using an antibody or method described herein, and treating the subject, for example using an antibody or method described herein. The invention also relates to an antibody, a combination of antibodies, or a pharmaceutical composition according to the invention for use in a method of a treating coronavirus (e.g. SARS-CoV-2) infection, as described herein, or a disease or complication associated therewith, e.g. COVID-19.

The invention also relates to a method of formulating a composition for treating coronavirus (e.g. SARS-CoV-2) infections, a disease or complication associated therewith, e.g. COVID-19, wherein said method comprises mixing an antibody, a combination of antibodies, or a pharmaceutical composition according to the invention with an acceptable carrier to prepare said composition.

The invention also relates to the use of an antibody, a combination of antibodies, or a pharmaceutical composition according to the invention for the manufacture of a medicament for treating a coronavirus (e.g. SARS-CoV-2) infection, as described herein, or a disease or complication associated therewith, e.g. COVID-19.

The invention also relates to identifying subjects that have a coronavirus infection, such as by SARS-CoV-2. For example, the methods and uses of the invention may involve identifying the presence of coronavirus (e.g. SARS-CoV-2), or a protein or a protein fragment thereof, in a sample. The detection may be carried out *in vitro* or *in vivo.* In certain embodiments, the invention relates to population screening. The invention relates to identifying any suitable SARS-CoV-2 strain, for example as described herein.

The methods and uses of the invention may include contacting a suitable sample from a subject, e.g. a blood, serum, mucus, saliva or other sample as described herein, with an antibody or a combination of antibodies of the invention, and detecting the presence or absence of an antibody-antigen complex, wherein the presence of the antibody-antigen complex indicates that the subject is infected with SARS-CoV-2.

Methods of determining the presence of an antibody-antigen complex are known in the art. For example, in vitro detection techniques include enzyme linked immunosorbent assays (ELISAs), Western blots, immunoprecipitations, and immunofluorescence. In vivo techniques include introducing into a subject a labelled anti-analyte protein antibody. For example, the antibody can be labelled with a radioactive marker whose presence and location in a subject can be detected by standard imaging techniques. The detection techniques may provide a qualitative or a quantitative readout depending on the assay employed.

Typically, the invention relates to methods and uses for a human subject in need thereof. However, non-human animals such as rats, rabbits, sheep, pigs, cows, cats, or dogs is also contemplated.

The subject may be asymptomatic or pre-symptomatic. The subject may be early, middle or late phase of the disease. The subject may be male or female. In certain embodiments, the subject is typically male. The subject may not have been infected with coronavirus, such as SARS-CoV-2. In embodiments of the invention relating to prevention or treatment, the subject may or may not have been diagnosed to be infected with coronavirus, such as SARS-CoV-2.

The invention relates to analysing samples from subjects. The sample may be tissues, cells and biological fluids isolated from a subject, as well as tissues, cells and fluids present within a subject. The sample may be blood and a fraction or component of blood including blood serum, blood plasma, or lymph. Typically, the sample is from a throat swab, nasal swab, or saliva. The antibody-antigen complex detection assays may be performed *in situ,* in which case the sample may be a tissue section (fixed and/or frozen) of the tissue obtained from biopsies or resections from a subject.

Antibodies, antibody combinations or pharmaceutical compositions may be administered subcutaneous, intravenous, intradermal, oral, intranasal, intramuscular or intracranial. Typically, administration is intravenous or subcutaneous.

The dose of an antibody may vary depending on the age and size of a subject, as well as on the disease, conditions and route of administration. Antibodies may be administered at a dose of about 0.1 mg/kg body weight to a dose of about 100 mg/kg body weight, such as at a dose of about 5 mg/kg to about 10 mg/kg. Antibodies may also be administered at a dose of about 50 mg/kg, 10 mg/kg or about 5 mg/kg body weight.

A combination of the invention may for example be administered at a dose of about 5 mg/kg to about 10 mg/kg for each antibody, or at a dose of about 10 mg/kg or about 5 mg/kg for each antibody. Alternatively, a combination may be administered at a dose of about 5 mg/kg total (e.g. a dose of 1.25 mg/kg of each antibody for a four antibody combination).

The antibody or combination of antibodies of the invention may be administered in a multiple dosage regimen. For example, the initial dose may be followed by administration of a second or plurality of subsequent doses. The second and subsequent doses may be separated by an appropriate time.

The antibodies of the invention are typically used in a single pharmaceutical composition/combination (co-formulated). However, the invention also generally includes the combined use of antibodies of the invention in separate preparations/compositions/ administrations. The invention also includes combined use of the antibodies with additional therapeutic agents, as described above.

Combined administration of the two or more agents and/or antibodies may be achieved in a number of different ways. In one embodiment, all the components may be administered together in a single composition. In another embodiment, each component may be administered separately as part of a combined therapy. For example, the antibody of the invention may be administered before, after or concurrently with another antibody of the invention. For example, the antibody of the invention may be administered before, after or concurrently with an anti-viral agent or an anti-inflammatory agent.

In embodiments where the invention relates to detecting the presence of coronavirus, e.g. SARS-CoV-2, or a protein or a protein fragment thereof, in a sample, the antibody may contain a detectable label. Methods of attaching a label to an antibody are known in the art, e.g. by direct labelling of the antibody by coupling (i.e., physically linking) a detectable substance to the antibody. Alternatively, the antibody may be indirectly labelled, e.g. by reactivity with another reagent that is directly labelled. Examples of indirect labelling include detection of a primary antibody using a fluorescently-labelled secondary antibody and end-labelling of a DNA probe with biotin such that it can be detected with fluorescently-labelled streptavidin.

The detection may further comprise: (i) an agent known to be useful for detecting the presence of coronavirus, e.g. SARS-CoV-2, or a protein or a protein fragment thereof, e.g. an antibody against other epitopes of the spike protein, or other proteins of the coronavirus, such as an anti-nucleocapsid antibody; and/or (ii) an agent known to not be capable of detecting the presence of coronavirus, e.g. SARS-CoV-2, or a protein or a protein fragment thereof, i.e. providing a negative control.

In certain embodiments, the antibody is modified to have increased stability. Suitable modifications are known in the art.

The invention also encompasses kits for detecting the presence of coronavirus, e.g. SARS-CoV-2, in a sample. For example, the kit may comprise: a labelled antibody or a combination of labelled antibodies of the invention; means for determining the amount of coronavirus, e.g. SARS-CoV-2, in a sample; and means for comparing the amount of coronavirus, e.g. SARS-CoV-2, in the sample with a standard. The labelled antibody or the combination of labelled antibodies can be packaged in a suitable container. The kit can further comprise instructions for using the kit to detect coronavirus, e.g. SARS-CoV-2, in a sample. The kit may further comprise other agents known to be useful for detecting the presence of coronavirus, as discussed above.

For example, the antibodies or combinations of antibodies of the invention may be used in a lateral flow test. Typically, the lateral flow test kit is a hand-held device with an absorbent pad, which based on a series of capillary beds, such as pieces of porous paper, microstructured polymer, or sintered polymer. The test runs the liquid sample along the surface of the pad with reactive molecules that show a visual positive or negative result. The test may further comprise using other agents known to be useful for detecting the presence of coronavirus, e.g. SARS-CoV-2, or a protein or a protein fragment thereof, as discussed above, such as anti- an anti-nucleocapsid antibody.

### Other

The present disclosure includes the combination of the aspects and features described except where such a combination is clearly impermissible or is stated to be expressly avoided. As used in this specification and the claims, the singular forms "a", "an", and "the" include plural referents unless the content clearly dictates otherwise. Thus, for example, reference to "an antibody" includes two or more such antibodies.

Section headings are used herein for convenience only and are not to be construed as limiting in any way.

When referring to "≥$x$" herein, this means equal to or greater than $x$. When referred to "≤$x$" herein, this means less than or equal to $x$.

For the purpose of this invention, in order to determine the percent identity of two sequences (such as two antibody sequences), the sequences are aligned for optimal comparison purposes (*e.g.*, gaps can be introduced in a first sequence for optimal alignment with a second sequence). The amino acids at each position are then compared. When a position in the first sequence is occupied by the same amino acid as the corresponding position in the second sequence, then the amino acids are identical at that position. The percent identity between the two sequences is a function of the number of identical positions shared by the sequences (*i.e.*, % identity = number of identical positions /total number of positions in the reference sequence x 100).

Typically the sequence comparison is carried out over the length of the reference sequence, for example, SEQ ID NO: 2081 herein. If the sequence is shorter than the reference sequence, the gaps or missing positions should be considered to be non-identical positions.

The skilled person is aware of different computer programs that are available to determine the homology or identity between two sequences using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6. Other examples of suitable programs are the BESTFIT program provided by the UWGCG Package (for example used on its default settings) (Devereux et al (1984) Nucleic Acids Research 12, 387-395) and the PILEUP and BLAST algorithms c (for example used on its default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (http://www.ncbi.nlm.nih.gov/).

The amino acid position numberings provided herein used the IMGT numbering system), although in some instances the KABAT numbering system or the absolute numbering of the amino acids based on the sequence listing may be used.

All publications, patents and patent applications cited herein, whether supra or infra, are hereby incorporated by reference in their entirety.

The following examples illustrate the invention.

## Examples

Example 1

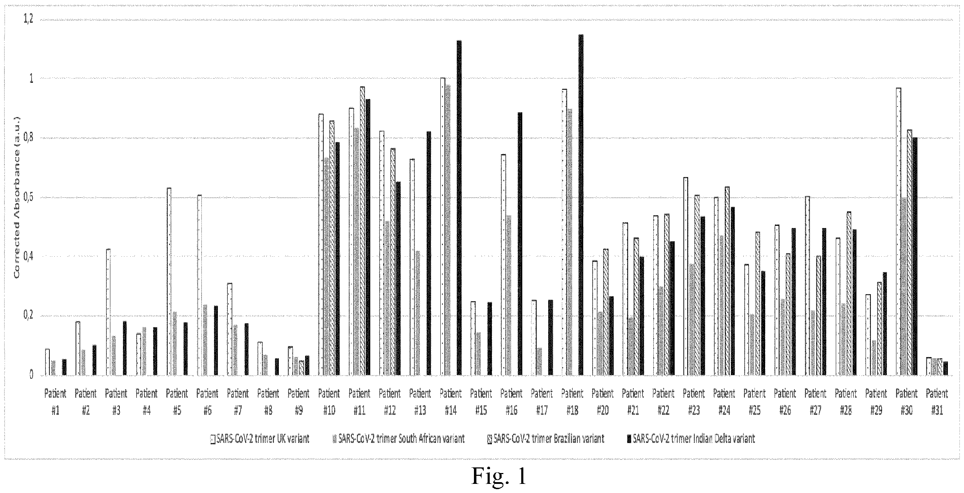

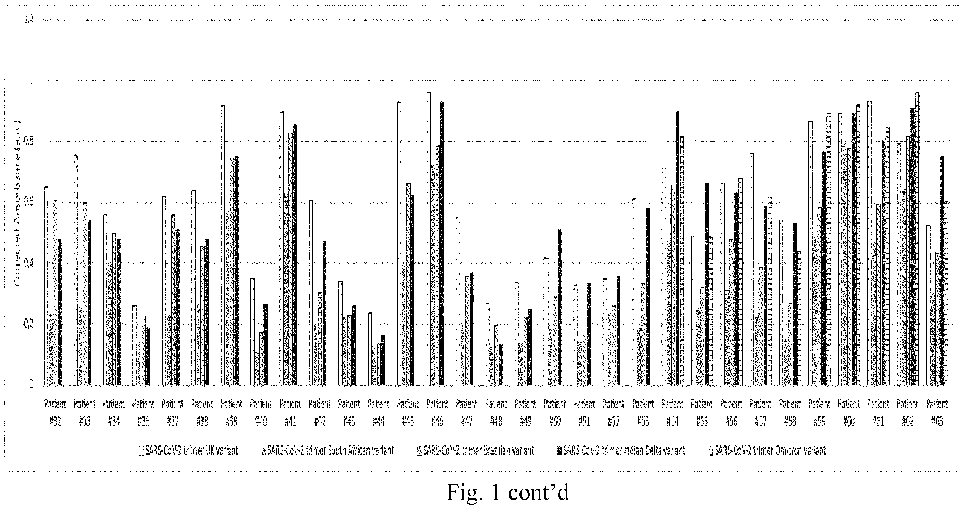

Receptor binding domain (RBD) and spike protein (S 1) of SARS-CoV-2 were used for B-cell selection since these domains are crucial antibody binding domains. Sixty-two convalescent Covid-19 patients (Table 1) were selected based on a recent history of SARS-CoV-2 infection, and/or has been vaccinated against SARS-CoV-2, and is suspected to have neutralizing antibodies against SARS-CoV-2 with a high affinity against SARS-CoV-2, that are cross-reactive against older SARS-CoV strains. From these patients, 50 mL of blood was withdrawn and tested for the presence of neutralizing antibodies (cPass). Furthermore, the specificity of the antibodies in blood serum for different SARS-CoV-2 variants was tested using ELISA (Figure 1). B-cells were isolated from the blood and SARS-CoV-2 binding antibodies were selected using FACS. After isolation, the B-cells were labelled with anti-CD27, which is a marker for memory B-cells, and SARS-CoV-2 Spike RBD or S 1 labelled with PE and FITC. Cells positive for CD27 and Spike were sorted (10/well) in a 96 well plate in the presence of irradiated EL4B5 cells, which function as feeder cells. In total 8203 wells were cultured with sorted B-cells from 54 patients. After 11 days the culture medium was harvested, and the cells were lysed and stored for further analysis.

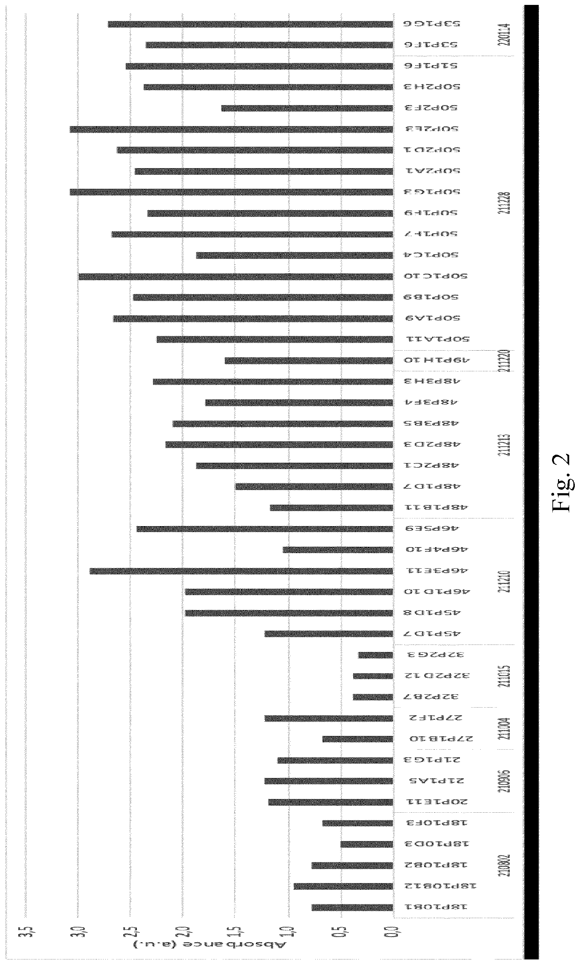

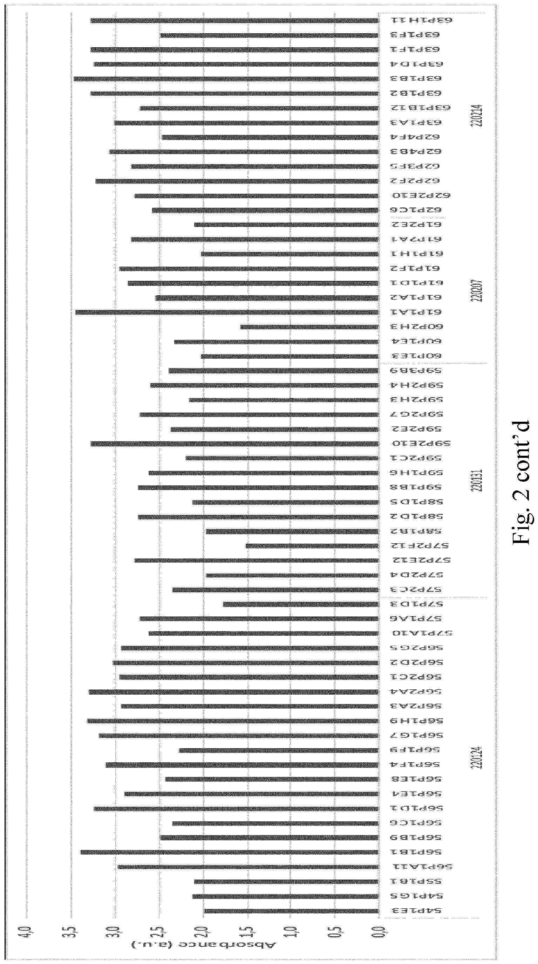

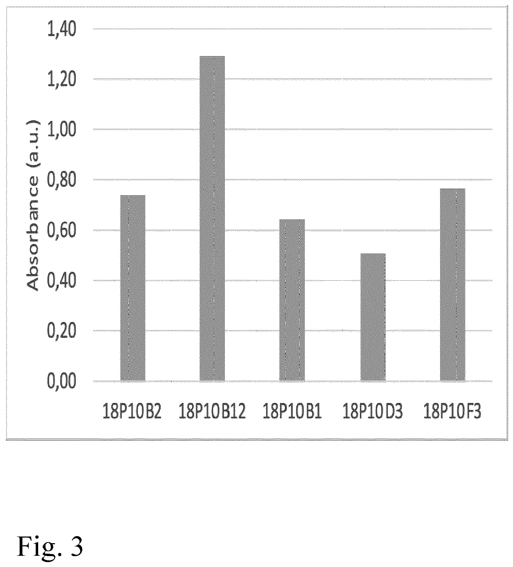

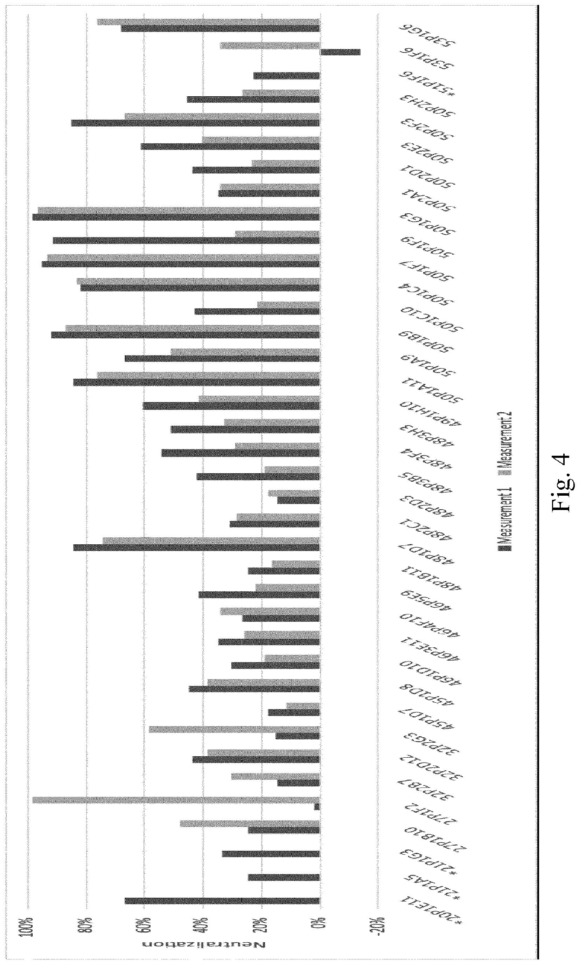

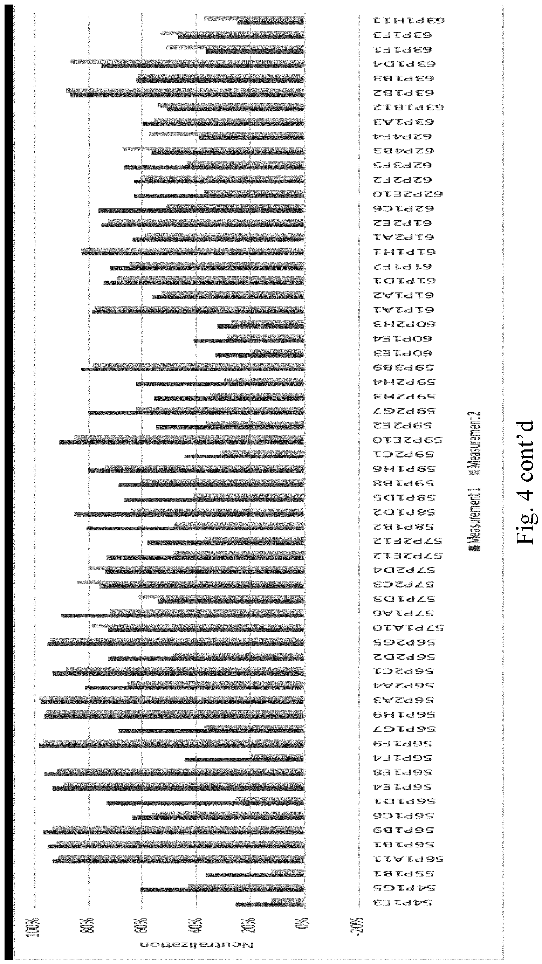

The culture medium from the B-cells was screened for the presence of antibodies using a capture ELISA (Figure 2). A little over 41% of the wells showed high antibody production, followed by 10% for medium and 9% showing low production. Culture medium containing antibodies (high and medium, some low) was further tested for the presence of SARS-CoV-2 binding/ neutralizing antibodies. Of 819 wells the antibodies were tested using target ELISA (Figure 3). Another part (1770 wells) was tested using a neutralization assay (Figure 4). When specific antibodies were present, the lyzed B-cells were sequenced and the most prominent antibody sequences were chosen to be produced in-house. Unique sequences were sub-cloned into antibody expression vectors. A total of 103 unique VH sequences were found. For the light chain, 34 unique lambda and 73 unique kappa sequences were found [predominant VL, VK]. From the 130 antibodies produced, 40 contain a lambda light chain and 90 a kappa light chain. Gene analysis of the subset of selected clones on SARS-CoV-2 showed that 5 germline families VH1, VH2, VH3, VH4 and VH5 were used for the variable heavy chain domain of the human IgG1, VHs originating form from 8 alleles from the VH1 family, originating from 1 alleles from the VH2 family, 14 allele from the VH3 family, 3 allele for from the VH4 family, and 2 from the VH5 family. For the light chains it showed that 10 germline genes were used, VL1, VL2, VL3, VL6, VL7, VL8, VK1(D), VK2, VK3 and VK4. VKs originated from 18 alleles and VL from 13 alleles.

Example 2

After expression of the human IgG1 antibodies containing the found VH and VL or VK, the antibodies (1 μg/mL) were tested for binding to spike protein virus variants using ELISA. Antibodies were tested for binding to Delta and Omicron. To test for non-specific binding BSA was included. For the first set of 62 antibodies, 41 antibodies were found to bind to Delta and/or Omicron (Figure 5, Absorbance > 0.5 a.u.). The remaining 21 did not show binding to either spike protein.

Example 3

A spike neutralization assay was performed to determine whether the binding antibodies were able to interfere with the interaction between the ACE2 receptor expressed on CHO-S cells and a PE-labelled Omicron SARS-CoV-2 spike protein. Five antibodies (Ab754, Ab759, Ab761, Ab763 and Ab772) showed high neutralization levels which did not even drop below 50% neutralization at the lowest concentration tested. Another set of 9 antibodies showed poorer neutralization, but still outperformed a previously developed neutralizing antibody Ab391. Ab753 showed slightly weaker neutralization than Ab391. Ab770 seems to partially neutralize the omicron spike protein. The remaining 25 antibodies show less neutralization than Ab391 or no neutralization at all.

Example 4

Clones that showed good neutralization (16) were tested in an epitope binning experiment on the Octet96. From this effort clones were obtained that were able to bind the target simultaneously (Table 2). A total of 3 bins was found using antibodies which were previously found to bind in different bind (Ab371, Ab391 and Ab390). The best binders bind to bin Ab371 and bin Ab391.

Example 5

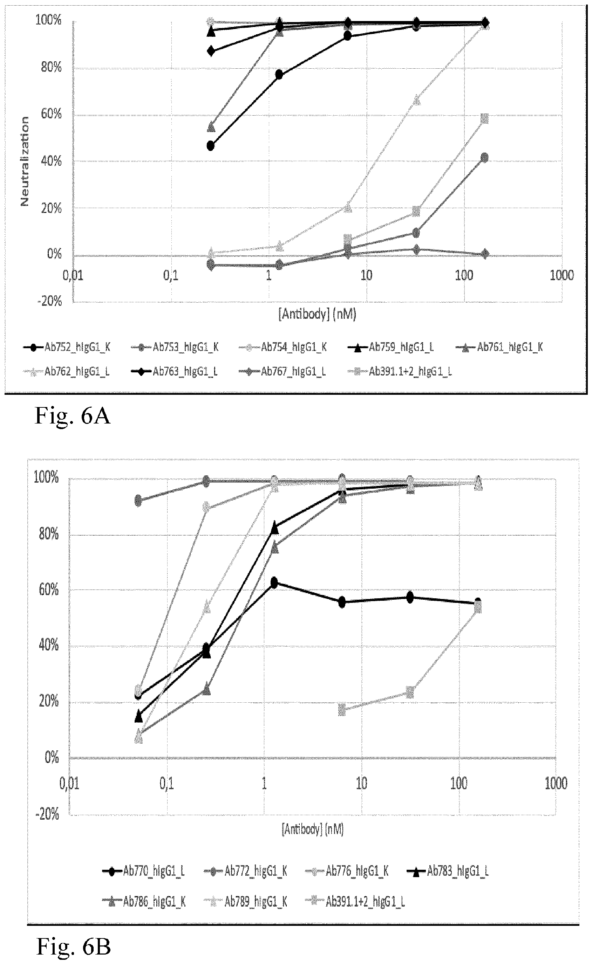

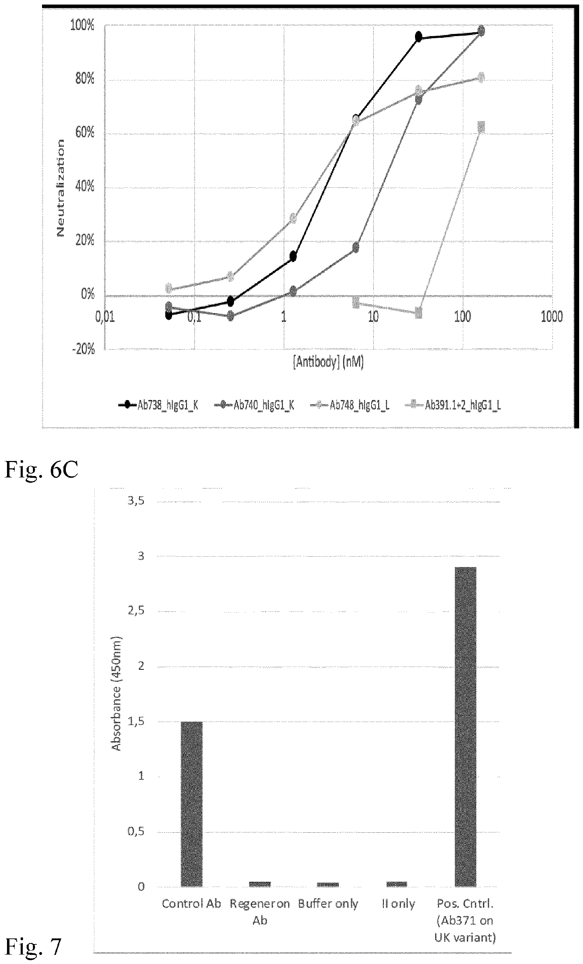

Binding studies were performed to compare binding of the "Standard Ab" (=Ab391 described in WO 2022/238481 and used in Examples 3 and 4, Figure 6 and Table 2 described above) and Regeneron antibody REGN10987 to Omikron variant (B.1.1.529) of the SARS-COV-2 virus. An ELISA assay showed that Ab391 binds to the Omikron variant, whilst REGN10987 does not (Figure 7).

**Materials and Methods**

*Study population*

The study population is an adult convalescent patient population that was infected with the SARS-CoV-2 virus, devoid of symptoms associated with an active SARS-CoV-2 infection, and/or has been vaccinated against SARS-CoV-2, and is suspected to have neutralizing antibodies against SARS-CoV-2 with a high affinity against SARS-CoV-2, that are cross-reactive against older SARS-CoV strains.

*Subject selection*

Subjects were selected based on a recent history of SARS-CoV-2 infection. A high level of neutralizing antibodies (measured using ePass™ (Genscript)) and high levels of antibodies against the S 1 antigen of SARS-CoV-2 (Euroimmun S 1 ELISA) were leading.

*Sample collection*

Every subject donated 50 mL of blood via venipuncture. Part of the sample was to determine the presence of neutralizing antibodies using the cPass surrogate virus neutralization test (GenScript) by Innatoss Laboratories B.V. The remainder was transferred to AbSano.

*Sample processing*

The blood was processed the day of the withdrawal. The different blood products were separated by centrifugation with Ficoll.
First the blood was diluted by adding 2 volumes of PBS+0,38% Na-citrate to 1 volume of blood. Next Ficoll Paque™ PLUS was added and the samples were centrifuged for 25 minutes at 800 xg (No brakes). The Plasma layer was collected for serum ELISA and the remainder stored at -80 °C. The peripheral blood mononuclear cells (PBMCs) layer was collected and used for B-cell isolation. The red blood cells and Ficol layer was discarded.

*Serum ELISA*

The proteins were coated at 2 $\mu$g/mL (SARS-CoV-2 Spike Trimer/ BSA) or 1 $\mu$g/mL (SARS-CoV-2 Spike RBD) in Carbonate buffer (pH 9.6) in a 96-well high bind ELISA plate (Greiner bio-one, cat.#655061) overnight at 4 °C. After washing the plate 3 times with PBS, the plate was blocked using a 1 w/v% BSA in PBS solution (B-PBS) for 1 hour. Serum samples were diluted 100x in PBS and subsequently a 2-step 10-fold dilution series in PBS was made. After washing the blocked plate 3 times with PBS, the wells were incubated with 100 $\mu$L of the diluted serum for 1 hour. After washing 3x PBST, a 5000x dilution of anti-hIgG antibody-HRP (ITK Diagnostics B.V., cat.#2010-05) in PBST was added and incubated for 1 hour. After washing 3x PBST and 3x PBS, the plate was developed using 50 $\mu$l/well TMB substrate according to the manufacturer's recommendation and the absorbance was recorded after stopping the reaction by adding 50 $\mu$l/well 2 M $H_2SO_4$ at 450 nm on a Tecan Infinite F50 plate reader.

*B-cell isolation, sorting and culturing*

First the PMBCs derived from the blood samples were washed twice in PBS+0,38% Na-citrate and the cells were counted. Next, B-cells were isolated using the B Cell Isolation Kit II, human (Miltenyi Biotec) according to the manufacturer's instructions. After the isolation the samples contained enriched B-cells in 1 ml cold PBE buffer (sterile PBS+0.5%BSA+2mM EDTA). From these cells 0,1×10^6 cells were stored in lysis buffer for sequencing by GenXPro and 0,8×10^6 cells were centrifuged, and the cell pellet was stored at -80 °C for library generation. The remainder of the cells were used for B-cell sorting. First, cells were stained for 1 hour using a mix or anti-CD27-APC, Spike-FITC and Spike-PE in PBE buffer. For the spike conjugates, biotinylated-RBD or S1 protein were pre-labelled with streptavidin-PE (Miltenyi Biotec, 130-106-790) or streptavidin-FITC (Miltenyi Biotec, 130-106-932). After the incubation, the cells were washed in PBE buffer. CD27$^+$Spike-PE$^+$Spike-FITC$^+$ cells were sorted using a Sony SH800S and deposited per 10 into a 96 well plate containing 20,000 feeder cells (irradiated EL4B5 cells with 2500 rad Cesium-137) in 200 $\mu$L 1 % hTSN + 5% BCS in DMEM/F12 complete medium (DMEM/F12 + 2 mM Glutamax + 2.3 mg/L $\beta$-mercaptoethanol + 1.22 mg/L Ethanolamine + 0.45 $\mu$g/L Sodium Selenite+ Pen/strep). Cells were cultures at 37 °C and 5% CO$_2$ for 11 days. On day 3 and day 6 half of the medium (100 $\mu$L) is replaced with 100 $\mu$L fresh 0.75 % hTSN + 5 % BCS in DMEM/F12 complete medium. On day 11, 180 $\mu$L of the medium is collected and stored in a 96 well plate at 4 °C for antibody screening. The remaining cells in the plates are lysed by addition of 150 $\mu$L Standard GenXPro Lysis buffer and stored at -80 °C until send for sequencing by GenXPro.

*Capture ELISA*

Overnight 96-wells plates were coated with 100 $\mu$L of 0.5 $\mu$g/mL goat anti-human lambda-UNLB + 0.5 $\mu$g/mL goat anti-human kappa-UNLB in PBS at 4 °C. Next, the plates were washed with 0.05% Tween-20 in PBS (PBST) and for 1 hour using a 1 w/v% BSA in PBST solution (B-PBST). After washing with 3 times with PBST, wells were filled with 80 $\mu$L B-PBST and 20 $\mu$L medium from the B-cell culture and incubated for 1 hour of incubation at 37 °C. After washing 3x PBST, a 5000x dilution of anti-hIgG antibody-HRP (ITK Diagnostics B.V., cat.#2010-05) in B-PBST was added and incubated for 1 hour at 37 °C. After washing 3x PBST and 3x PBS, the plate was developed using 50 $\mu$l/well TMB substrate according to the manufacturer's recommendation and the absorbance was recorded after stopping the reaction by adding 50 $\mu$l/well 2 M H$_2$SO$_4$ at 450 nm on a Tecan Infinite F50 plate reader. Cells showing antibody production were further analysed.

*Target ELISA*

*Neutralization assay*

Spike neutralization assays were used to determine the antibody neutralization. A Biotin-labelled spike protein, Indian Delta - Spike (B.1.617.2) and Omicron - Trimer (B.1.1.529), were labelled with a streptavidin-PE label. The PE-labelled spike was then incubated with B-cell culture medium from antibody expressing wells in a 1:1 ratio for one hour. After incubation 50 $\mu$L of the mixture was added to a mixture of encoded hACE2-expressing CHO-S cells and non-expressing CHO-S cells (100,000 cells each) and incubated for 1 hour. After washing the fluorescence of the cells was recorded using a flow cytometer (Beckman Coulter, CytoFlex). Neutralization per sample was calculated using formula 1 (see below).

$$1 - \frac{percentage\ PE^+\ hACE2\ cells\ with\ antibody}{percentage\ PE^+\ hACE2\ cells\ no\ antibody}\ (\text{Formula 1})$$

Duplo's, recorded starting from patient 27, were calculated separately.

For expressed antibodies, the antibodies (51,2 pM - 160 nM) were used directly after harvest, in cell culture medium. Concentration of antibodies was determined using BLI (Forte Bio, Octet96) and single measurements were done to determine the neutralization.

*Re-cloning into mammalian expression vectors*

The antibody inserts, VL or VH, were orded at Thermo Fisher Scientific in a pMA-RQ vector surrounded by BsmI (Bioké, cat.#R0134) and BsiWI (Bioké, cat.#R0553) restriction sites. After digestion the insert was ligated into the corresponding linearized vector (pABS-hIgG1, pABS-hkappa or pABS-hlambda) using T4 DNA ligase. All sequences were verified via Sanger sequencing (Macrogen Europe B.V.) using specific primer for the pABS plasmids (5'-AGGGAGACCCAAGCTAG -3', Eurogentec) and analysed with CLC main workbench software.

*Cell culture*

HEK293F cells (Invitrogen) were cultured in suspension in FreestyleTM 293 expression medium (Thermo Fisher Scientific, cat.#12338) at 37 °C, 110 rpm, 8 % CO2 and 95 % humidity. Cells were sub-cultured 3x-week at 0.3-0.5 million/mL in culture flasks. For counting and viability check an automated cell counter (Nexcelom Cellometer auto T4 Plus) was used and cells were diluted 1:1 in 0.4% Trypan Blue (Thermo Fisher Scientific, cat.#15250061).

*Antibody expression*

Plasmids encoding for the antibodies were amplified in chemically competent *E. coli* XL1-blue bacteria and harvested using Mini/Midi/Maxi prep (Qiagen, cat.#27106, cat.#27191, cat.#12143; Sigma-Aldrich, cat.#NA0410). HEK293F cells (1 million/mL) were transfected with FectoPro transfection reagent (PolyPlus-transfection, cat.#116) 333 $\mu$g DNA/million cells of light chain plasmid and 167 $\mu$g/million cells of heavy chain plasmid for full size antibody expression. After 2.5 hours, transfection was boosted by addition of 2 mM Sodium butyrate. Cells were incubated at 37 °C, 110 rpm, 8 % $CO_2$ and 95 % humidity. After 4-6 days the cells were checked for viability and the antibody was harvested when the viability dropped below 60%, as detected using automated cell counting. To harvest the antibody, the culture was first centrifuged at 300 xg for 10 minutes at 4 °C to remove the cells and next at 4816 xg for 1 hour at 4 °C to remove cell debris and aggregates. The supernatant, containing the antibody in culture medium, was stored at 4 °C until further use. Antibody concentration was assessed using Octet measurements (ForteBio Octet Red96) with a Protein A coated sensor (Molecular Devices, cat.#18-5010) and a hIgG1 standard curve (Sartorius, cat.#18-1118). Typical concentrations ranged between 0.01 and 0.4 mg/mL.

*Epitope binning*

To identify antibodies with considerable similar or overlapping epitopes a cross competition assay was performed. Epitope binning was conducted using an in-tandem format involving the immobilization of 25 nM biotinylated RBD (Bio-connect, #40592-V08H-B) on streptavidin biosensors which are subsequently presented to the two competing antibodies. First, the RBD-loaded sensor was dipped into 100 nM of first antibody until flattening of the binding curve was visible. The first antibodies were taken from Antibodies patent (PCT/EP2022/062777) and all bind to different epitopes. The sensors with antigen-antibody 1 were then incubated in the second antibody (50 nM) supplemented with 100 nM antibody 1, for 120 seconds. In each round, also binding without first or second antibody was recorded. For data analysis, if a second antibody still binds RDB which has been pre-captured by a first antibody, the antibodies are defined as non-overlapping epitopes ("+"); if the second antibody does not bind the RBD pre-captured by the first antibody the antibodies are competitive ("-"). Antibody pairs with competition ("-") are grouped in the same bin.

## Table 1: Patient information

| Patient nr. | Date PCR+ or start of symptoms | Detection | Vaccine | Vaccination date (1) | Vaccination date (2) | Vaccination date (3) | Date blood collection | cPass | S1-ELISA |
|---|---|---|---|---|---|---|---|---|---|
| 1 | 21-Mar-21 | PCR | No | | | | 1-Jul-21 | 0,43 | 0,9 |
| 2 | 12-May-21 | PCR | No | | | | 1-Jul-21 | 0,59 | 3,4 |
| 3 | 14-Apr-21 | PCR | No | | | | 1-Jul-21 | 0,98 | 7,3 |
| 4 | 18-May-21 | PCR | No | | | | 1-Jul-21 | 0,21 | 0,4 |
| 5 | 3-Jun-21 | PCR | No | | | | 8-Jul-21 | 0,92 | 8,9 |

| | | | | | | | | | |
|---|---|---|---|---|---|---|---|---|---|
| 6 | 15-May-21 | PCR | No | | | | 8-Jul-21 | 0,92 | 8,6 |
| 7 | 1-Apr-21 | PCR | No | | | | 8-Jul-21 | 0,64 | 5,2 |
| 8 | April 2021 | Antibody test | No | | | | 8-Jul-21 | no sample | |
| 9 | 6-Jun-21 | PCR | No | | | | 14-Jul-21 | 0,24 | |
| 10 | 26-Mar-20 | Antibody test Innatoss | Pfizer (1x) | May 2021 | | | 14-Jul-21 | 0,97 | |
| 11 | 23-Feb-20 | Antibody test Innatoss | Moderna (1x) | Unkown | | | 14-Jul-21 | 0,98 | |
| 12 | 9-Mar-20 | Antibody test Innatoss | Pfizer (2x) | 8-May-21 | 12-Jun-21 | | 14-Jul-21 | 0,97 | |
| 13 | 15-Mar-20 | Antibody test Innatoss | AstraZeneca (2x) | 30-Apr-21 | 23-Jun-21 | | 20-Jul-21 | 0,98 | |
| 14 | March 2020 | Antibody test | Pfizer (2x) | 11-May-21 | 15-Jun-21 | | 20-Jul-21 | | |
| 15 | Non | Non | AstraZeneca (2x) | Unknown | 17-Jul-21 | | 20-Jul-21 | 0,83 | |
| 16 | March 2020 + March 2021 | Antibody test Innatoss | Pfizer (2x) | 16-May-21 | 20-Jun-21 | | 22-Jul-21 | 0,98 | |
| 17 | March 2020 | Antibody test Innatoss | AstraZeneca (2x) | 30-Mar-21 | 15-Jul-21 | | 22-Jul-21 | 0,96 | |
| 18 | 16-Mar-20 | Antibody test Innatoss | Pfizer (2x) | 31-May-21 | 6-Jul-21 | | 22-Jul-21 | 0,98 | |
| 19 | Not included | | | | | | | | |
| 20 | Non | Non | Pfizer (2x) | 4-Jul-21 | 7-Aug-21 | | 24-Aug-21 | | |
| 21 | 23-Feb-21 | PCR | mRNA vaccine (1x) | Unkown | | | 24-Aug-21 | 0,97 | |
| 22 | Non | Non | Pfizer (2x) | 23-Jun-21 | 28-Jul-21 | | 26-Aug-21 | | |
| 23 | 23-Feb-21 | PCR | Pfizer (1x) | 17-Jun-21 | | | 26-Aug-21 | 0,97 | |
| 24 | 15-May-21 | PCR | mRNA vaccine (1x) | Unkown | | | 16-Sep-21 | 0,96 | |
| 25 | 23-Feb-21 | PCR | mRNA vaccine (1x) | Unkown | | | 16-Sep-21 | 0,97 | |
| 26 | 2020 | PCR | Yes | Unkown | | | 21-Sep-21 | 0,97 | |
| 27 | 3-Jun-21 | PCR | Yes | Unkown | | | 21-Sep-21 | 0,96 | |
| 28 | 17-Mar-20 | Antibody test Innatoss | Yes | Unkown | | | 23-Sep-21 | 0,93 | |
| 29 | 13-Jan-21 | PCR | mRNA vaccine (1x) | 21-Jun-21 | | | 23-Sep-21 | 0,96 | |
| 30 | 9-Mar-20 | Antibody test Innatoss | mRNA vaccine (1x) | 25-Jul-21 | | | 28-Sep-21 | 0,24 | |
| 31 | 5-May-21 | PCR | Pfizer (1x) | 23-Jun-21 | | | 28-Sep-21 | 0,97 | |
| 32 | 23-Mar-20 | Antibody test Innatoss | mRNA vaccine (2x) | Unkown | Unkown | | 5-Oct-21 | 0,97 | |
| 33 | 16-Apr-21 | PCR | Pfizer (1x) | 4-Jun-21 | | | 5-Oct-21 | 0,97 | |

| | | | | | | | | |
|---|---|---|---|---|---|---|---|---|
| 34 | Spring 2020 | Antibody test Innatoss | mRNA vaccine (2x) | Unkown | Unkown | | 12-Oct-21 | 0,07 | |
| 35 | Spring 2020 | Antibody test Innatoss | mRNA vaccine (2x) | Unkown | Unkown | | 12-Oct-21 | 0,84 | |
| 36 | 10-May-21 | PCR | Pfizer (2x) | 13-Jun-21 | 19-Jul-21 | | 12-Oct-21 | 0,96 | |
| 37 | 16-Mar-20 | Antibody test Innatoss | mRNA vaccine (2x) | Unkown | Unkown | | 26-Oct-21 | 0,95 | |
| 38 | 16-Mar-20 | Antibody test Innatoss | mRNA vaccine (2x) | Unkown | Unkown | | 26-Oct-21 | 0,97 | |
| 39 | Spring 2021 | Antibody test Innatoss | mRNA vaccine (2x) | Unkown | Unkown | | 2-Nov-21 | 0,97 | |
| 40 | Spring 2021 | PCR | mRNA vaccine (2x) | Unkown | Unkown | | 2-Nov-21 | 0,96 | |
| 41 | 21-Mar-20 | Antibody test Innatoss | mRNA vaccine (2x) | Unkown | Unkown | | 4-Nov-21 | 0,97 | |
| 42 | Non | Non | Pfizer (2x) | 24-Jun-21 | 29-Jul-21 | | 04-Nov-21 | | |
| 43 | 3-Jun-21 | PCR | Pfizer (1x) | 29-Jul-21 | | | 16-Nov-21 | 0,95 | |
| 44 | Mar-May 2021 | PCR | mRNA vaccine (1x) | Unkown | | | 16-Nov-21 | 0,93 | |
| 45 | February 2020 | Antibody test Innatoss | mRNA vaccine (2x) | Unkown | Unkown | | 30-Nov-21 | 0,97 | |
| 46 | 13-Nov-21 | PCR | AstraZeneca (2x) | 4-May-21 | 22-Jun-21 | | 30-Nov-21 | 0,98 | |
| 47 | 15-Sep-21 | PCR | Pfizer (2x) | 19-Apr-21 | 26-May-21 | | 2-Dec-21 | 0,97 | |
| 48 | 16-Apr-21 | PCR | Pfizer (2x) | 10-Jun-21 | 18-Jul-21 | | 2-Dec-21 | 0,97 | |
| 49 | 18-Apr-21 | PCR | Pfizer (2x) | 21-May-21 | 18-Jul-21 | | 7-Dec-21 | 0,97 | |
| 50 | Fall 2021 | PCR | Pfizer (2x) | 7-May-21 | 11-Jun-21 | | 16-Dec-21 | 0,97 | |
| 51 | 10-Nov-21 | PCR | Pfizer (2x) | 22-Jun-21 | 17-Jul-21 | | 16-Dec-21 | 0,98 | |
| 52 | 14-Aug-21 | PCR | AstraZeneca (2x) | 4-Mar-21 | 20-May-21 | | 4-Jan-22 | 0,92 | |
| 53 | 11-Nov-21 | PCR | Pfizer (2x) | 24-Jun-21 | 29-Jul-21 | | 4-Jan-22 | | 11,3 |
| 54 | 6-Dec-21 | PCR | Pfizer (2x) | 19-Jun-21 | 24-Jul-21 | | 11-Jan-22 | | 13,5 |
| 55 | 24-Sep-21 | PCR | Pfizer (2x) | 30-Jun-21 | 4-Aug-21 | | 11-Jan-22 | | 11,5 |
| 56 | 29-Dec-21 | PCR | Pfizer (2x) | 19-Jun-21 | 23-Jul-21 | | 13-Jan-22 | 0,89 | |
| 57 | 30-Dec-21 | PCR | Pfizer (2x) | 2-Jun-21 | 7-Jul-21 | | 13-Jan-22 | 0,97 | |
| 58 | 3-Jan-22 | PCR | mRNA vaccine (2x) | Unkown | Unkown | | 20-Jan-22 | 0,97 | |
| 59 | 3-Jan-22 | PCR | mRNA vaccine (2x) | Unkown | Unkown | | 20-Jan-22 | 0,97 | |
| 60 | 7-Jan-22 | PCR | Moderna (2x) + Pfizer (booster) | 15-Apr-21 | 10-May-21 | 24-Nov-21 | 27-Jan-22 | 0,97 | |
| 61 | 8-Jan-22 | PCR | mRNA vaccine (2x) | 11-May-21 | 16-Jun-21 | | 27-Jan-22 | 0,97 | |
| 62 | 22-Nov-21 | PCR | mRNA vaccine (2x) | 13-May-21 | 17-06-2021 | | 3-Feb-22 | 0,97 | |
| 63 | 18-Dec-21 | PCR | mRNA vaccine (2x) | Unkown | Unkown | | 3-Feb-22 | 0,96 | |

Table 2: Epitope binning

| Ab2 \ Ab1 | Ab371_hIgG1_L | Ab391_hIgG1_L | Ab390_ScFv_VL1 |
|---|---|---|---|
| Ab752_hIgG1_K | - | + | + |
| Ab754_hIgG1_K | - | + | + |
| Ab759_hIgG1_L | - | + | + |
| Ab763_hIgG1_L | - | + | + |
| Ab772_hIgG1_K | - | + | + |
| Ab776_hIgG1_K | - | + | + |
| Ab783_hIgG1_L | - | + | + |
| Ab786_hIgG1_K | - | +/- | + |
| Ab789_hIgG1_K | - | +/- | + |
| Ab738_hIgG1_K | + | - | + |
| Ab740_hIgG1_K | + | - | + |
| Ab762_hIgG1_L | + | - | + |
| Ab748_hIgG1_L | + | + | - |
| Ab753_hIgG1_K | + | + | - |
| Ab770_hIgG1_L | + | + | - |
| Ab761_hIgG1_K | + | + | - |

(+) denotes additional binding of Ab2. (-) denotes no additional binding of Ab2, thus they belong to the same bin.

## Table 3 – Antibody Sequences

| Delta/ Omicron | Antibody ID No. / B-cell well | | VH Sequence | VH CDR1 | VH CDR2 | VH CDR3 | VL Sequence | VL CDR1 | VL CDR2 | | VL CDR3 | VH Framework 1 | VH Framework 2 | VH Framework 3 | VH Framework 4 | VL Framework 1 | VL Framework 2 | VL Framework 3 | VL Framework 4 |
|---|---|---|---|---|---|---|---|---|---|---|---|---|---|---|---|---|---|---|---|
| Delta | Ab704 | VH18P10B2 | 1 | 2 | 3 | 4 | 5 | 6 | 7 | RTN | 8 | 1041 | 1042 | 1043 | 1044 | 1045 | 1046 | 1047 | 1048 |
| Delta | Ab705 | VH18P10B12 | 9 | 10 | 11 | 12 | 13 | 14 | 15 | DAS | 16 | 1049 | 1050 | 1051 | 1052 | 1053 | 1054 | 1055 | 1056 |
| Delta | Ab706 | VH18P10B1.A | 17 | 18 | 19 | 20 | 21 | 22 | 23 | GNN | 24 | 1057 | 1058 | 1059 | 1060 | 1061 | 1062 | 1063 | 1064 |
| Delta | Ab707 | VH18P10B1.A | 25 | 26 | 27 | 28 | 29 | 30 | 31 | EVS | 32 | 1065 | 1066 | 1067 | 1068 | 1069 | 1070 | 1071 | 1072 |
| Delta | Ab708 | VH18P10B1.B | 33 | 34 | 35 | 36 | 37 | 38 | 39 | GNN | 40 | 1073 | 1074 | 1075 | 1076 | 1077 | 1078 | 1079 | 1080 |
| Delta | Ab709 | VH18P10B1.B | 41 | 42 | 43 | 44 | 45 | 46 | 47 | EVS | 48 | 1081 | 1082 | 1083 | 1084 | 1085 | 1086 | 1087 | 1088 |
| Delta | Ab710 | VH18P10D3.A | 49 | 50 | 51 | 52 | 53 | 54 | 55 | AAS | 56 | 1089 | 1090 | 1091 | 1092 | 1093 | 1094 | 1095 | 1096 |
| Delta | Ab711 | VH18P10D3.A | 57 | 58 | 59 | 60 | 61 | 62 | 63 | GAS | 64 | 1097 | 1098 | 1099 | 1100 | 1101 | 1102 | 1103 | 1104 |
| Delta | Ab712 | VH18P10D3.B | 65 | 66 | 67 | 68 | 69 | 70 | 71 | AAS | 72 | 1105 | 1106 | 1107 | 1108 | 1109 | 1110 | 1111 | 1112 |
| Delta | Ab713 | VH18P10D3.B | 73 | 74 | 75 | 76 | 77 | 78 | 79 | GAS | 80 | 1113 | 1114 | 1115 | 1116 | 1117 | 1118 | 1119 | 1120 |
| Delta | Ab738_hIgG1_K | VH18P10F3 | 81 | 82 | 83 | 84 | 85 | 86 | 87 | DAS | 88 | 1121 | 1122 | 1123 | 1124 | 1125 | 1126 | 1127 | 1128 |
| Delta | Ab739_hIgG1_K | VH20P1E11 | 89 | 90 | 91 | 92 | 93 | 94 | 95 | AAS | 96 | 1129 | 1130 | 1131 | 1132 | 1133 | 1134 | 1135 | 1136 |
| Delta | Ab740_hIgG1_K | VH21P1G3 | 97 | 98 | 99 | 100 | 101 | 102 | 103 | GAS | 104 | 1137 | 1138 | 1139 | 1140 | 1141 | 1142 | 1143 | 1144 |
| Delta | Ab742_hIgG1_L | VH21P1A5 | 105 | 106 | 107 | 108 | 109 | 110 | 111 | EGN | 112 | 1145 | 1146 | 1147 | 1148 | 1149 | 1150 | 1151 | 1152 |
| Delta | Ab744_hIgG1_L | VH27P1F2 | 113 | 114 | 115 | 116 | 117 | 118 | 119 | EVS | 120 | 1153 | 1154 | 1155 | 1156 | 1157 | 1158 | 1159 | 1160 |
| Delta | Ab746_hIgG1_L | VH27P1B10 | 121 | 122 | 123 | 124 | 125 | 126 | 127 | EDN | 128 | 1161 | 1162 | 1163 | 1164 | 1165 | 1166 | 1167 | 1168 |
| Delta | Ab747_hIgG1_K | VH32P2B7.A | 129 | 130 | 131 | 132 | 133 | 134 | 135 | GAS | 136 | 1169 | 1170 | 1171 | 1172 | 1173 | 1174 | 1175 | 1176 |
| Delta | Ab748_hIgG1_L | VH32P2B7.A | 137 | 138 | 139 | 140 | 141 | 142 | 143 | EVS | 144 | 1177 | 1178 | 1179 | 1180 | 1181 | 1182 | 1183 | 1184 |
| Delta | Ab749_hIgG1_K | VH32P2B7.B | 145 | 146 | 147 | 148 | 149 | 150 | 151 | GAS | 152 | 1185 | 1186 | 1187 | 1188 | 1189 | 1190 | 1191 | 1192 |
| Delta | Ab750_hIgG1_L | VH32P2B7.B | 153 | 154 | 155 | 156 | 157 | 158 | 159 | EVS | 160 | 1193 | 1194 | 1195 | 1196 | 1197 | 1198 | 1199 | 1200 |
| Delta | Ab751_hIgG1_K | VH32P2D12 | 161 | 162 | 163 | 164 | 165 | 166 | 167 | GAS | 168 | 1201 | 1202 | 1203 | 1204 | 1205 | 1206 | 1207 | 1208 |

| | | | 169 | 170 | 171 | 172 | 173 | 174 | 175 | | 176 | 1209 | 1210 | 1211 | 1212 | 1213 | 1214 | 1215 | 1216 |
|---|---|---|---|---|---|---|---|---|---|---|---|---|---|---|---|---|---|---|---|
| Delta | Ab752_hIgG1_K | VH46P5E9 | 169 | 170 | 171 | 172 | 173 | 174 | 175 | GAS | 176 | 1209 | 1210 | 1211 | 1212 | 1213 | 1214 | 1215 | 1216 |
| Delta | Ab753_hIgG1_K | VH46P3H11 | 177 | 178 | 179 | 180 | 181 | 182 | 183 | KVS | 184 | 1217 | 1218 | 1219 | 1220 | 1221 | 1222 | 1223 | 1224 |
| Delta | Ab754_hIgG1_K | VH46P4F10 | 185 | 186 | 187 | 188 | 189 | 190 | 191 | DAS | 192 | 1225 | 1226 | 1227 | 1228 | 1229 | 1230 | 1231 | 1232 |
| Delta | Ab756_hIgG1_L | VH48P1B11 | 193 | 194 | 195 | 196 | 197 | 198 | 199 | DYS | 200 | 1233 | 1234 | 1235 | 1236 | 1237 | 1238 | 1239 | 1240 |
| Delta | Ab757_hIgG1_K | VH48P2C1 | 201 | 202 | 203 | 204 | 205 | 206 | 207 | WAS | 208 | 1241 | 1242 | 1243 | 1244 | 1245 | 1246 | 1247 | 1248 |
| Delta | Ab758_hIgG1_L | VH48P3H3 | 209 | 210 | 211 | 212 | 213 | 214 | 215 | RNN | 216 | 1249 | 1250 | 1251 | 1252 | 1253 | 1254 | 1255 | 1256 |
| Delta | Ab759_hIgG1_L | VH48P3B5 | 217 | 218 | 219 | 220 | 221 | 222 | 223 | DNN | 224 | 1257 | 1258 | 1259 | 1260 | 1261 | 1262 | 1263 | 1264 |
| Delta | Ab760_hIgG1_K | VH49P1H10 | 225 | 226 | 227 | 228 | 229 | 230 | 231 | WAS | 232 | 1265 | 1266 | 1267 | 1268 | 1269 | 1270 | 1271 | 1272 |
| Delta | Ab761_hIgG1_K | VH50P1A9 | 233 | 234 | 235 | 236 | 237 | 238 | 239 | GAS | 240 | 1273 | 1274 | 1275 | 1276 | 1277 | 1278 | 1279 | 1280 |
| Delta | Ab762_hIgG1_L | VH50P1B9 | 241 | 242 | 243 | 244 | 245 | 246 | 247 | GNS | 248 | 1281 | 1282 | 1283 | 1284 | 1285 | 1286 | 1287 | 1288 |
| Delta | Ab763_hIgG1_L | VH50P2F3 | 249 | 250 | 251 | 252 | 253 | 254 | 255 | DNQ | 256 | 1289 | 1290 | 1291 | 1292 | 1293 | 1294 | 1295 | 1296 |
| Delta | Ab764_hIgG1_K | VH45P1D7.A | 257 | 258 | 259 | 260 | 261 | 262 | 263 | AAS | 264 | 1297 | 1298 | 1299 | 1300 | 1301 | 1302 | 1303 | 1304 |
| Delta | Ab765_hIgG1_L | VH45P1D7.A | 265 | 266 | 267 | 268 | 269 | 270 | 271 | NTI | 272 | 1305 | 1306 | 1307 | 1308 | 1309 | 1310 | 1311 | 1312 |
| Delta | Ab766_hIgG1_K | VH45P1D7.B | 273 | 274 | 275 | 276 | 277 | 278 | 279 | AAS | 280 | 1313 | 1314 | 1315 | 1316 | 1317 | 1318 | 1319 | 1320 |
| Delta | Ab767_hIgG1_L | VH45P1D7.B | 281 | 282 | 283 | 284 | 285 | 286 | 287 | NTI | 288 | 1321 | 1322 | 1323 | 1324 | 1325 | 1326 | 1327 | 1328 |
| Delta | Ab768_hIgG1_K | VH45P1D8 | 289 | 290 | 291 | 292 | 293 | 294 | 295 | SAS | 296 | 1329 | 1330 | 1331 | 1332 | 1333 | 1334 | 1335 | 1336 |
| Delta | Ab769_hIgG1_K | VH46P1D10 | 297 | 298 | 299 | 300 | 301 | 302 | 303 | KAS | 304 | 1337 | 1338 | 1339 | 1340 | 1341 | 1342 | 1343 | 1344 |
| Delta | Ab770_hIgG1_L | VH48P3F4 | 305 | 306 | 307 | 308 | 309 | 310 | 311 | DNT | 312 | 1345 | 1346 | 1347 | 1348 | 1349 | 1350 | 1351 | 1352 |
| Delta | Ab771_hIgG1_K | VH50P1C4 | 313 | 314 | 315 | 316 | 317 | 318 | 319 | KAS | 320 | 1353 | 1354 | 1355 | 1356 | 1357 | 1358 | 1359 | 1360 |
| Delta | Ab772_hIgG1_K | VH50P1F7 | 321 | 322 | 323 | 324 | 325 | 326 | 327 | GAS | 328 | 1361 | 1362 | 1363 | 1364 | 1365 | 1366 | 1367 | 1368 |
| Delta | Ab773_hIgG1_K | VH50P1H9 | 329 | 330 | 331 | 332 | 333 | 334 | 335 | WAS | 336 | 1369 | 1370 | 1371 | 1372 | 1373 | 1374 | 1375 | 1376 |
| Delta | Ab774_hIgG1_K | VH50P1C10 | 337 | 338 | 339 | 340 | 341 | 342 | 343 | AAS | 344 | 1377 | 1378 | 1379 | 1380 | 1381 | 1382 | 1383 | 1384 |
| Delta | Ab775_hIgG1_L | VH50P1A11.A | 345 | 346 | 347 | 348 | 349 | 350 | 351 | DVS | 352 | 1385 | 1386 | 1387 | 1388 | 1389 | 1390 | 1391 | 1392 |
| Delta | Ab776_hIgG1_K | VH50P1A11.A | 353 | 354 | 355 | 356 | 357 | 358 | 359 | VAS | 360 | 1393 | 1394 | 1395 | 1396 | 1397 | 1398 | 1399 | 1400 |
| Delta | Ab777_hIgG1_L | VH50P1A11.B | 361 | 362 | 363 | 364 | 365 | 366 | 367 | DVS | 368 | 1401 | 1402 | 1403 | 1404 | 1405 | 1406 | 1407 | 1408 |
| Delta | Ab778_hIgG1_K | VH50P1A11.B | 369 | 370 | 371 | 372 | 373 | 374 | 375 | VAS | 376 | 1409 | 1410 | 1411 | 1412 | 1413 | 1414 | 1415 | 1416 |
| Delta | Ab779_hIgG1_K | VH50P2H3 | 377 | 378 | 379 | 380 | 381 | 382 | 383 | KAS | 384 | 1417 | 1418 | 1419 | 1420 | 1421 | 1422 | 1423 | 1424 |
| Delta | Ab780_hIgG1_K | VH51P1F6 | 385 | 386 | 387 | 388 | 389 | 390 | 391 | AAS | 392 | 1425 | 1426 | 1427 | 1428 | 1429 | 1430 | 1431 | 1432 |

EP 4 385 999 A1

| Delta | Ab781_hIgG1_K | VH50P1G3 | 393 | 394 | 395 | 396 | 397 | 398 | 399 | KAS | 400 | 1433 | 1434 | 1435 | 1436 | 1437 | 1438 | 1439 | 1440 |
|---|---|---|---|---|---|---|---|---|---|---|---|---|---|---|---|---|---|---|---|---|
| Delta | Ab782_hIgG1_L | VH32P2G3 | 401 | 402 | 403 | 404 | 405 | 406 | 407 | DVS | 408 | 1441 | 1442 | 1443 | 1444 | 1445 | 1446 | 1447 | 1448 |
| Delta | Ab783_hIgG1_L | VH50P2A1 | 409 | 410 | 411 | 412 | 413 | 414 | 415 | DDS | 416 | 1449 | 1450 | 1451 | 1452 | 1453 | 1454 | 1455 | 1456 |
| Delta | Ab784_hIgG1_K | VH50P2D1 | 417 | 418 | 419 | 420 | 421 | 422 | 423 | DAS | 424 | 1457 | 1458 | 1459 | 1460 | 1461 | 1462 | 1463 | 1464 |
| Delta | Ab785_hIgG1_K | VII50P2E3.A | 425 | 426 | 427 | 428 | 429 | 430 | 431 | AAS | 432 | 1465 | 1466 | 1467 | 1468 | 1469 | 1470 | 1471 | 1472 |
| Delta | Ab786_hIgG1_K | VH50P2E3.Λ | 433 | 434 | 435 | 436 | 437 | 438 | 439 | GAT | 440 | 1473 | 1474 | 1475 | 1476 | 1477 | 1478 | 1479 | 1480 |
| Delta | Ab787_hIgG1_K | VH50P2E3.B | 441 | 442 | 443 | 444 | 445 | 446 | 447 | AAS | 448 | 1481 | 1482 | 1483 | 1484 | 1485 | 1486 | 1487 | 1488 |
| Delta | Ab788_hIgG1_K | VH50P2E3.B | 449 | 450 | 451 | 452 | 453 | 454 | 455 | GAT | 456 | 1489 | 1490 | 1491 | 1492 | 1493 | 1494 | 1495 | 1496 |
| Delta | Ab789_hIgG1_K | VH48P1D7 | 457 | 458 | 459 | 460 | 461 | 462 | 463 | AAS | 464 | 1497 | 1498 | 1499 | 1500 | 1501 | 1502 | 1503 | 1504 |
| Delta | Ab790_hIgG1_K | VH48P2D3.A | 465 | 466 | 467 | 468 | 469 | 470 | 471 | DAS | 472 | 1505 | 1506 | 1507 | 1508 | 1509 | 1510 | 1511 | 1512 |
| Delta | Ab791_hIgG1_K | VH48P2D3.A | 473 | 474 | 475 | 476 | 477 | 478 | 479 | GAS | 480 | 1513 | 1514 | 1515 | 1516 | 1517 | 1518 | 1519 | 1520 |
| Delta | Ab792_hIgG1_K | VH48P2D3.B | 481 | 482 | 483 | 484 | 485 | 486 | 487 | DAS | 488 | 1521 | 1522 | 1523 | 1524 | 1525 | 1526 | 1527 | 1528 |
| Delta | Ab793_hIgG1_K | VH48P2D3.B | 489 | 490 | 491 | 492 | 493 | 494 | 495 | GAS | 496 | 1529 | 1530 | 1531 | 1532 | 1533 | 1534 | 1535 | 1536 |
| Delta | | VH53P1F6 | 497 | 498 | 499 | 500 | 501 | 502 | 503 | KDS | 504 | 1537 | 1538 | 1539 | 1540 | 1541 | 1542 | 1543 | 1544 |
| Delta | | VH53P1G6 | 505 | 506 | 507 | 508 | 509 | 510 | 511 | SNN | 512 | 1545 | 1546 | 1547 | 1548 | 1549 | 1550 | 1551 | 1552 |
| Omicron | | VH54P1E3 | 513 | 514 | 515 | 516 | 517 | 518 | 519 | SVS | 520 | 1553 | 1554 | 1555 | 1556 | 1557 | 1558 | 1559 | 1560 |
| Omicron | | VH54P1G5 | 521 | 522 | 523 | 524 | 525 | 526 | 527 | KAS | 528 | 1561 | 1562 | 1563 | 1564 | 1565 | 1566 | 1567 | 1568 |
| Omicron | | VH55P1B1 | 529 | 530 | 531 | 532 | 533 | 534 | 535 | GAS | 536 | 1569 | 1570 | 1571 | 1572 | 1573 | 1574 | 1575 | 1576 |
| Delta | | VH56P1B1 | 537 | 538 | 539 | 540 | 541 | 542 | 543 | VNS | 544 | 1577 | 1578 | 1579 | 1580 | 1581 | 1582 | 1583 | 1584 |
| Delta | | VH56P1D1-E4 | 545 | 546 | 547 | 548 | 549 | 550 | 551 | GAS | 552 | 1585 | 1586 | 1587 | 1588 | 1589 | 1590 | 1591 | 1592 |
| Delta | | VH56P1D1-E4 | 553 | 554 | 555 | 556 | 557 | 558 | 559 | DAS | 560 | 1593 | 1594 | 1595 | 1596 | 1597 | 1598 | 1599 | 1600 |
| Delta | | VH56P1F4 | 561 | 562 | 563 | 564 | 565 | 566 | 567 | TTS | 568 | 1601 | 1602 | 1603 | 1604 | 1605 | 1606 | 1607 | 1608 |
| Delta | | VH56P1C6 | 569 | 570 | 571 | 572 | 573 | 574 | 575 | AAS | 576 | 1609 | 1610 | 1611 | 1612 | 1613 | 1614 | 1615 | 1616 |
| Delta | | VH56P1G7 | 577 | 578 | 579 | 580 | 581 | 582 | 583 | QDT | 584 | 1617 | 1618 | 1619 | 1620 | 1621 | 1622 | 1623 | 1624 |
| Delta | | VH56P1E8 | 585 | 586 | 587 | 588 | 589 | 590 | 591 | GAS | 592 | 1625 | 1626 | 1627 | 1628 | 1629 | 1630 | 1631 | 1632 |
| Delta | | VH56P1B9 | 593 | 594 | 595 | 596 | 597 | 598 | 599 | GAS | 600 | 1633 | 1634 | 1635 | 1636 | 1637 | 1638 | 1639 | 1640 |
| Delta | | VII56P1F9 | 601 | 602 | 603 | 604 | 605 | 606 | 607 | AAS | 608 | 1641 | 1642 | 1643 | 1644 | 1645 | 1646 | 1647 | 1648 |
| Delta | | VH56P1H9 | 609 | 610 | 611 | 612 | 613 | 614 | 615 | AAS | 616 | 1649 | 1650 | 1651 | 1652 | 1653 | 1654 | 1655 | 1656 |
| Delta | | VH56P1A11 | 617 | 618 | 619 | 620 | 621 | 622 | 623 | AAS | 624 | 1657 | 1658 | 1659 | 1660 | 1661 | 1662 | 1663 | 1664 |

25

| 1672 | 1671 | 1670 | 1669 | 1668 | 1667 | 1666 | 1665 | 632 | | 631 | 630 | 629 | 628 | 627 | 626 | 625 | Name | Variant |
|---|---|---|---|---|---|---|---|---|---|---|---|---|---|---|---|---|---|---|
| 1672 | 1671 | 1670 | 1669 | 1668 | 1667 | 1666 | 1665 | 632 | AAS | 631 | 630 | 629 | 628 | 627 | 626 | 625 | VH56P2C1 | Omicron |
| 1680 | 1679 | 1678 | 1677 | 1676 | 1675 | 1674 | 1673 | 640 | KDT | 639 | 638 | 637 | 636 | 635 | 634 | 633 | VH56P2D2 | Omicron |
| 1688 | 1687 | 1686 | 1685 | 1684 | 1683 | 1682 | 1681 | 648 | EAS | 647 | 646 | 645 | 644 | 643 | 642 | 641 | VH56P2A3 | Omicron |
| 1696 | 1695 | 1694 | 1693 | 1692 | 1691 | 1690 | 1689 | 656 | EDN | 655 | 654 | 653 | 652 | 651 | 650 | 649 | VH56P2A4 | Omicron |
| 1704 | 1703 | 1702 | 1701 | 1700 | 1699 | 1698 | 1697 | 664 | KDS | 663 | 662 | 661 | 660 | 659 | 658 | 657 | VH56P2G5-57P2D4-F12 | Omicron |
| 1712 | 1711 | 1710 | 1709 | 1708 | 1707 | 1706 | 1705 | 672 | AAS | 671 | 670 | 669 | 668 | 667 | 666 | 665 | VH57P1D3 | Delta |
| 1720 | 1719 | 1718 | 1717 | 1716 | 1715 | 1714 | 1713 | 680 | AAS | 679 | 678 | 677 | 676 | 675 | 674 | 673 | VH57P1A6 | Delta |
| 1728 | 1727 | 1726 | 1725 | 1724 | 1723 | 1722 | 1721 | 688 | WAS | 687 | 686 | 685 | 684 | 683 | 682 | 681 | VH57P1A10 | Delta |
| 1736 | 1735 | 1734 | 1733 | 1732 | 1731 | 1730 | 1729 | 696 | AAS | 695 | 694 | 693 | 692 | 691 | 690 | 689 | VH57P2C3 | Omicron |
| 1744 | 1743 | 1742 | 1741 | 1740 | 1739 | 1738 | 1737 | 704 | AAS | 703 | 702 | 701 | 700 | 699 | 698 | 697 | VH56P2G5-57P2D4-F12 | Omicron |
| 1752 | 1751 | 1750 | 1749 | 1748 | 1747 | 1746 | 1745 | 712 | KAS | 711 | 710 | 709 | 708 | 707 | 706 | 705 | VH57P2E12 | Omicron |
| 1760 | 1759 | 1758 | 1757 | 1756 | 1755 | 1754 | 1753 | 720 | NNN | 719 | 718 | 717 | 716 | 715 | 714 | 713 | VH56P2G5-57P2D4-F12 | Omicron |
| 1768 | 1767 | 1766 | 1765 | 1764 | 1763 | 1762 | 1761 | 728 | DAS | 727 | 726 | 725 | 724 | 723 | 722 | 721 | VH58P1B2 | Omicron |
| 1776 | 1775 | 1774 | 1773 | 1772 | 1771 | 1770 | 1769 | 736 | AAS | 735 | 734 | 733 | 732 | 731 | 730 | 729 | VH58P1D2 | Omicron |
| 1784 | 1783 | 1782 | 1781 | 1780 | 1779 | 1778 | 1777 | 744 | DAS | 743 | 742 | 741 | 740 | 739 | 738 | 737 | VH58P1D5 | Omicron |
| 1792 | 1791 | 1790 | 1789 | 1788 | 1787 | 1786 | 1785 | 752 | TAS | 751 | 750 | 749 | 748 | 747 | 746 | 745 | VH59P1H6 | Delta |
| 1800 | 1799 | 1798 | 1797 | 1796 | 1795 | 1794 | 1793 | 760 | NAS | 759 | 758 | 757 | 756 | 755 | 754 | 753 | VH59P1R8 | Delta |
| 1808 | 1807 | 1806 | 1805 | 1804 | 1803 | 1802 | 1801 | 768 | AAS | 767 | 766 | 765 | 764 | 763 | 762 | 761 | VH59P2C1 | Omicron |
| 1816 | 1815 | 1814 | 1813 | 1812 | 1811 | 1810 | 1809 | 776 | GAS | 775 | 774 | 773 | 772 | 771 | 770 | 769 | VH59P2E2 | Omicron |
| 1824 | 1823 | 1822 | 1821 | 1820 | 1819 | 1818 | 1817 | 784 | GGS | 783 | 782 | 781 | 780 | 779 | 778 | 777 | VH59P2H3 | Omicron |
| 1832 | 1831 | 1830 | 1829 | 1828 | 1827 | 1826 | 1825 | 792 | TLS | 791 | 790 | 789 | 788 | 787 | 786 | 785 | VH59P2H4 | Omicron |
| 1840 | 1839 | 1838 | 1837 | 1836 | 1835 | 1834 | 1833 | 800 | AAS | 799 | 798 | 797 | 796 | 795 | 794 | 793 | VH59P2G7-60P1E4 | Omicron |
| 1848 | 1847 | 1846 | 1845 | 1844 | 1843 | 1842 | 1841 | 808 | FVI | 807 | 806 | 805 | 804 | 803 | 802 | 801 | VH59P2G7-60P1F4 | Omicron |
| 1856 | 1855 | 1854 | 1853 | 1852 | 1851 | 1850 | 1849 | 816 | AAS | 815 | 814 | 813 | 812 | 811 | 810 | 809 | VH59P2E10 | Omicron |
| 1864 | 1863 | 1862 | 1861 | 1860 | 1859 | 1858 | 1857 | 824 | DAS | 823 | 822 | 821 | 820 | 819 | 818 | 817 | VH59P3B9 | Delta |
| 1872 | 1871 | 1870 | 1869 | 1868 | 1867 | 1866 | 1865 | 832 | DAS | 831 | 830 | 829 | 828 | 827 | 826 | 825 | VH60P1E3 | Delta |
| 1880 | 1879 | 1878 | 1877 | 1876 | 1875 | 1874 | 1873 | 840 | DDS | 839 | 838 | 837 | 836 | 835 | 834 | 833 | VH60P1E3 | Delta |
| 1888 | 1887 | 1886 | 1885 | 1884 | 1883 | 1882 | 1881 | 848 | DAS | 847 | 846 | 845 | 844 | 843 | 842 | 841 | VH59P2G7-60P1E4 | Delta |
| 1896 | 1895 | 1894 | 1893 | 1892 | 1891 | 1890 | 1889 | 856 | DGA | 855 | 854 | 853 | 852 | 851 | 850 | 849 | VH60P2H3 | Omicron |

EP 4 385 999 A1

| Delta | | VH61P1A1 | 857 | 858 | 859 | 860 | 861 | 862 | 863 | AAS | 864 | 1897 | 1898 | 1899 | 1900 | 1901 | 1902 | 1903 | 1904 |
|---|---|---|---|---|---|---|---|---|---|---|---|---|---|---|---|---|---|---|---|
| Delta | | VII61P1D1 | 865 | 866 | 867 | 868 | 869 | 870 | 871 | DDD | 872 | 1905 | 1906 | 1907 | 1908 | 1909 | 1910 | 1911 | 1912 |
| Delta | | VH61P1H1 | 873 | 874 | 875 | 876 | 877 | 878 | 879 | AAS | 880 | 1913 | 1914 | 1915 | 1916 | 1917 | 1918 | 1919 | 1920 |
| Delta | | VH61P1A2 | 881 | 882 | 883 | 884 | 885 | 886 | 887 | DVT | 888 | 1921 | 1922 | 1923 | 1924 | 1925 | 1926 | 1927 | 1928 |
| Delta | | VH61P1F2-P2E2-62P2E10 | 889 | 890 | 891 | 892 | 893 | 894 | 895 | GAS | 896 | 1929 | 1930 | 1931 | 1932 | 1933 | 1934 | 1935 | 1936 |
| Omicron | | VH61P2A1 | 897 | 898 | 899 | 900 | 901 | 902 | 903 | WAS | 904 | 1937 | 1938 | 1939 | 1940 | 1941 | 1942 | 1943 | 1944 |
| Omicron | | VH61P2A1 | 905 | 906 | 907 | 908 | 909 | 910 | 911 | EVS | 912 | 1945 | 1946 | 1947 | 1948 | 1949 | 1950 | 1951 | 1952 |
| Omicron | | VH61P1F2-P2E2-62P2E10 | 913 | 914 | 915 | 916 | 917 | 918 | 919 | WAS | 920 | 1953 | 1954 | 1955 | 1956 | 1957 | 1958 | 1959 | 1960 |
| Delta | | VH61P1F2-P2E2-62P2E10 | 921 | 922 | 923 | 924 | 925 | 926 | 927 | EDN | 928 | 1961 | 1962 | 1963 | 1964 | 1965 | 1966 | 1967 | 1968 |
| Delta | | VH62P1C6 | 929 | 930 | 931 | 932 | 933 | 934 | 935 | WAS | 936 | 1969 | 1970 | 1971 | 1972 | 1973 | 1974 | 1975 | 1976 |
| Delta | | VH62P2F2 | 937 | 938 | 939 | 940 | 941 | 942 | 943 | WAS | 944 | 1977 | 1978 | 1979 | 1980 | 1981 | 1982 | 1983 | 1984 |
| Delta | | VH61P1F2-P2E2-62P2E10 | 945 | 946 | 947 | 948 | 949 | 950 | 951 | AAS | 952 | 1985 | 1986 | 1987 | 1988 | 1989 | 1990 | 1991 | 1992 |
| Omicron | | VH62P3F5 | 953 | 954 | 955 | 956 | 957 | 958 | 959 | WAS | 960 | 1993 | 1994 | 1995 | 1996 | 1997 | 1998 | 1999 | 2000 |
| Omicron | | VH62P4B3 | 961 | 962 | 963 | 964 | 965 | 966 | 967 | AAS | 968 | 2001 | 2002 | 2003 | 2004 | 2005 | 2006 | 2007 | 2008 |
| Omicron | | VH62P4F4-63P1B2 | 969 | 970 | 971 | 972 | 973 | 974 | 975 | GAS | 976 | 2009 | 2010 | 2011 | 2012 | 2013 | 2014 | 2015 | 2016 |
| Delta | | VH63P1F1 | 977 | 978 | 979 | 980 | 981 | 982 | 983 | AAS | 984 | 2017 | 2018 | 2019 | 2020 | 2021 | 2022 | 2023 | 2024 |
| Delta | | VH62P4F4-63P1B2 | 985 | 986 | 987 | 988 | 989 | 990 | 991 | GAS | 992 | 2025 | 2026 | 2027 | 2028 | 2029 | 2030 | 2031 | 2032 |
| Delta | | VH63P1A3 | 993 | 994 | 995 | 996 | 997 | 998 | 999 | GAS | 1000 | 2033 | 2034 | 2035 | 2036 | 2037 | 2038 | 2039 | 2040 |
| Delta | | VH63P1B3-D4-H11 | 1001 | 1002 | 1003 | 1004 | 1005 | 1006 | 1007 | GAS | 1008 | 2041 | 2042 | 2043 | 2044 | 2045 | 2046 | 2047 | 2048 |
| Delta | | VH63P1F3 | 1009 | 1010 | 1011 | 1012 | 1013 | 1014 | 1015 | GAS | 1016 | 2049 | 2050 | 2051 | 2052 | 2053 | 2054 | 2055 | 2056 |
| Delta | | VH63P1B3-D4-H11 | 1017 | 1018 | 1019 | 1020 | 1021 | 1022 | 1023 | SNN | 1024 | 2057 | 2058 | 2059 | 2060 | 2061 | 2062 | 2063 | 2064 |
| Omicron | | VH63P1B3-D4-H11 | 1025 | 1026 | 1027 | 1028 | 1029 | 1030 | 1031 | DAS | 1032 | 2065 | 2066 | 2067 | 2068 | 2069 | 2070 | 2071 | 2072 |
| Omicron | | VH63P1B12 | 1033 | 1034 | 1035 | 1036 | 1037 | 1038 | 1039 | SNN | 1040 | 2073 | 2074 | 2075 | 2076 | 2077 | 2078 | 2079 | 2080 |

**Claims**

1. An antibody, or an antigen-binding fragment thereof, that binds to the spike protein of coronavirus SARS-CoV-2, wherein the antibody comprises a set of three heavy chain complementarity determining regions (CDRH1, CDRH2 and CDRH3) and three heavy chain complementarity determining regions (CDRL1, CDRL2 and CDRL3), wherein the set of CDRH1, CDRH2, CDRH3, CDRL1, CDRL2 and CDRL3 amino acid sequences is selected from:

   (a) SEQ ID NOs: 186, 187, 188, 190, 191 and 192;
   (b) SEQ ID NOs: 322, 323, 324, 326, 327 and 328;
   (c) SEQ ID NOs: 218, 219, 220, 222, 223 and 224;
   (d) SEQ ID NOs: 250, 251, 252, 254, 255 and 256;
   (e) SEQ ID NOs: 234, 235, 236, 238, 239 and 240;
   (f) SEQ ID NOs: 82, 83, 84, 86, 87 and 88;
   (g) SEQ ID NOs: 170, 171, 172, 174, 175 and 176;
   (h) SEQ ID NOs: 354, 355, 356, 358, 359 and 360;
   (i) SEQ ID NOs: 410, 411, 412, 414, 415 and 416;
   (j) SEQ ID NOs: 458, 459, 460, 462, 463 and 464;
   (k) SEQ ID NOs: 434, 435, 436, 438, 439 and 440;
   (l) SEQ ID NOs: 138, 139, 140, 142, 143 and 144;
   (m) SEQ ID NOs: 242, 243, 244, 246, 247 and 248;
   (n) SEQ ID NOs: 98, 99, 100, 102, 103 and 104;
   (o) SEQ ID NOs: 306, 307, 308, 310, 311 and 312; and
   (p) SEQ ID NOs: 178, 179, 180, 182, 183 and 184.

2. The antibody or fragment of claim 1, comprising

   (a) a heavy chain variable region having at least 80% amino acid sequence identity to any one of SEQ ID NOs: 185, 321, 217, 249, 233, 81, 169, 353, 409, 457, 433, 137, 241, 97, 305, and 177, or comprising the amino acid sequence of any one of SEQ ID NOs: 185, 321, 217, 249, 233, 81, 169, 353, 409, 457, 433, 137, 241, 97, 305, and 177; and/or
   (b) a light chain variable region having at least 80% amino acid sequence identity to any one of SEQ ID NOs: 189, 325, 221, 253, 237, 85, 173, 357, 413, 461, 437, 141, 245, 101, 309, and 181 or comprising the amino acid sequence of any one of SEQ ID NOs: 189, 325, 221, 253, 237, 85, 173, 357, 413, 461, 437, 141, 245, 101, 309, and 181.

3. The antibody or fragment of claim 1 or claim 2, that is a multivalent antibody, optionally wherein the multivalent antibody comprises a set of CDRH1, CDRH2, CDRH3, CDRL1, CDRL2 and CDRL3 amino acid sequences selected from (a) SEQ ID NOs: 186, 187, 188, 190, 191 and 192; (b) SEQ ID NOs: 322, 323, 324, 326, 327 and 328; (c) SEQ ID NOs: 218, 219, 220, 222, 223 and 224; (d) SEQ ID NOs: 250, 251, 252, 254, 255 and 256; (e) SEQ ID NOs: 234, 235, 236, 238, 239 and 240; and (f) SEQ ID NOs: 82, 83, 84, 86, 87 and 88.

4. The antibody or fragment of any one of claims 1 to 3, that is a multispecific antibody, optionally a bispecific antibody or a trispecific antibody, further optionally wherein the antibody of fragment comprises

   (A)

      i. one antigen-binding site comprising a set of CDRH1, CDRH2, CDRH3, CDRL1, CDRL2 and CDRL3 amino acid sequences selected from (I) SEQ ID NOs: 186, 187, 188, 190, 191 and 192, (II) SEQ ID NOs: 322, 323, 324, 326, 327 and 328, (III) SEQ ID NOs: 218, 219, 220, 222, 223 and 224, and (IV) SEQ ID NOs: 250, 251, 252, 254, 255 and 256;
      ii. one antigen-binding site comprising the set of CDRH1, CDRH2, CDRH3, CDRL1, CDRL2 and CDRL3 amino acid sequences of SEQ ID NOs: 82, 83, 84, 86, 87 and 88; and
      iii. one antigen-binding site comprising the set of CDRH1, CDRH2, CDRH3, CDRL1, CDRL2 and CDRL3 amino acid sequences of SEQ ID NOs: 234, 235, 236, 238, 239 and 240;

   (B)

      i. one antigen-binding site comprising a set of CDRH1, CDRH2, CDRH3, CDRL1, CDRL2 and CDRL3 amino acid sequences selected from (I) SEQ ID NOs: 186, 187, 188, 190, 191 and 192, (II) SEQ ID NOs: 322, 323, 324, 326, 327 and 328, (III) SEQ ID NOs: 218, 219, 220, 222, 223 and 224, and (IV) SEQ ID NOs: 250, 251, 252, 254, 255 and 256; and

ii. one antigen-binding site comprising the set of CDRH1, CDRH2, CDRH3, CDRL1, CDRL2 and CDRL3 amino acid sequences of SEQ ID NOs: 82, 83, 84, 86, 87 and 88;

(C)

i. one antigen-binding site comprising a set of CDRH1, CDRH2, CDRH3, CDRL1, CDRL2 and CDRL3 amino acid sequences selected from (I) SEQ ID NOs: 186, 187, 188, 190, 191 and 192, (II) SEQ ID NOs: 322, 323, 324, 326, 327 and 328, (III) SEQ ID NOs: 218, 219, 220, 222, 223 and 224, and (IV) SEQ ID NOs: 250, 251, 252, 254, 255 and 256; and

ii. one antigen-binding site comprising the set of CDRH1, CDRH2, CDRH3, CDRL1, CDRL2 and CDRL3 amino acid sequences of SEQ ID NOs: 234, 235, 236, 238, 239 and 240; or

(D)

i. one antigen-binding site the set of CDRH1, CDRH2, CDRH3, CDRL1, CDRL2 and CDRL3 amino acid sequences of SEQ ID NOs: 82, 83, 84, 86, 87 and 88; and

ii. one antigen-binding site comprising the set of CDRH1, CDRH2, CDRH3, CDRL1, CDRL2 and CDRL3 amino acid sequences of SEQ ID NOs: 234, 235, 236, 238, 239 and 240.

5. A combination of antibodies comprising two or more antibodies selected from the antibodies of claim 1(a) to (p).

6. The combination of antibodies of claim 5, comprising

(A) one or more antibodies selected from any two or more of the following groups (i) to (iii):

(i) the antibodies of claim 1 (a), (b), (c), (d), (g), (h), (i), (j) and (k);
(ii) the antibodies of claim 1 (f), (m) and (n); and
(iii) the antibodies of claim 1 (e), (1), (o) and (p); and/or

(B) two or more antibodies selected from the antibodies of claim 1 (a) to (f), optionally wherein

(i) the combination of antibodies comprises the antibody of claim 1(f), the antibody of claim 1(e), and any one of the antibodies of claim 1 (a) to (d);
(ii) the combination of antibodies comprises the antibody of claim 1(f) and any one of the antibodies of claim 1 (a) to (d);
(iii) the antibody of claim 1 (e) and any one of the antibodies of claim 1 (a) to (d); or
(iv) the combination of antibodies comprises the antibodies of claim 1 (e) and (f).

7. A pharmaceutical composition comprising the antibody according to any one of claims 1 to 4, or the combination of antibodies according to claim 5 or claim 6, and optionally at least one pharmaceutically acceptable diluent or carrier.

8. The antibody according to any one of claims 1 to 4, the combination of antibodies according to claim 5 or claim 6, or the pharmaceutical composition according to claim 7, for use in a method for treatment of a human or animal body by therapy.

9. The antibody according to any one of claims 1 to 4, the combination of antibodies according to claim 5 or claim 6, or the pharmaceutical composition according to claim 7, for use in a method of treating or preventing a coronavirus infection, or a disease or complication associated with coronavirus infection.

10. A method of treating a subject comprising administering a therapeutically effective amount of the antibody according to any one of claims 1 to 4, or the combination of antibodies according to claim 5 or claim 6, or the pharmaceutical composition according to claim 7, to the subject.

11. The method according to claim 10, wherein the method is for treating a coronavirus infection, or a disease or complication associated with coronavirus infection.

12. A method of identifying the presence of coronavirus, or a protein or a protein fragment thereof, in a sample, comprising:

(i) contacting the sample with an antibody according to any one of claims 1 to 4, or the combination of antibodies according to claim 5 or claim 6; and
(ii) detecting the presence or absence of an antibody-antigen complex,

wherein the presence of the antibody-antigen complex indicates the presence of coronavirus, or a protein or a protein fragment thereof, in the sample.

13. One or more polynucleotides encoding the antibody according to any one of claims 1 to 4, or the combination of antibodies according to claim 5 or claim 6.

14. One or more vectors comprising the polynucleotide or polynucleotides of claim 13 or a host cell comprising said vector or vectors.

15. A method for producing an antibody according to any one of claims 1 to 4, or the combination of antibodies according to claim 5 or claim 6, the method comprising culturing the host cell of claim 14 and isolating the antibody or antibodies from the culture.

Fig. 1

Fig. 1 cont'd

Fig. 2

Fig. 2 cont'd

Fig. 3

Fig. 4

■Measurement1 ▨Measurement:2

Fig. 4 cont'd

■Measurement 1  ▨Measurement 2

Neutralization

Fig. 5

Fig. 5 cont'd

Fig. 6A

Fig. 6B

Fig. 6C

Fig. 7

Europäisches
Patentamt

European
Patent Office

Office européen
des brevets

# EUROPEAN SEARCH REPORT

Application Number

EP 22 21 3518

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2022/238481 A1 (MODIQUEST B V [NL]) 17 November 2022 (2022-11-17) * claims 1,3,6,16,22,25-29; examples 1-17 * | 1-15 | INV. C07K16/10 |
| X | NIKITIN PAVEL A. ET AL: "IMM-BCP-01, a patient-derived anti-SARS-CoV-2 antibody cocktail, is active across variants of concern including Omicron BA.1 and BA.2", SCIENCE IMMUNOLOGY, vol. 7, no. 75, 9 September 2022 (2022-09-09), page eabl9943, XP93043321, DOI: 10.1126/sciimmunol.abl9943 * figures 1-5; tables 1-4 * | 1-15 | |
| X | US 2022/267415 A1 (KU ZHIQIANG [US] ET AL) 25 August 2022 (2022-08-25) * paragraph [0291] * | 1-15 | |
| X | WO 2022/184659 A1 (QUADRUCEPT BIO LTD [GB]) 9 September 2022 (2022-09-09) * examples 29-43 * | 1-15 | TECHNICAL FIELDS SEARCHED (IPC) C07K A61K |

The present search report has been drawn up for all claims

| Place of search | Date of completion of the search | Examiner |
|---|---|---|
| The Hague | 1 May 2023 | Lonnoy, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 22 21 3518

The Search Division considers that the present European patent application does not comply with the requirements of unity of invention and relates to several inventions or groups of inventions, namely:

**1. claims: 1-15**

An antibody that binds to the spike protein of coronavirus SARS-CoV-2, wherein the antibody comprises a set of three heavy chain complementarity determining regions (CDRH1, CDRH2 and CDRH3) and three heavy chain complementarity determining regions (CDRL1, CDRL2 and CDRL3); A combination of antibodies or pharmaceutical composition comprising said antibody; Said antibody for use in a method for treatment of a human or animal body by therapy; Said antibody for use in a method of treating or preventing a coronavirus infection, or a disease or complication associated with coronavirus infection; A method using said antibody for identifying the presence of coronavirus, or a protein or a protein fragment thereof, in a sample; A polynucleotide encoding said antibody; A vector or host cell comprising said polynucleotide; A method for producing said antibody comprising culturing said host cell

**1.1. claims: 1-15(partially)**

An antibody that binds to the spike protein of coronavirus SARS-CoV-2, wherein the antibody comprises a set of three heavy chain complementarity determining regions (CDRH1, CDRH2 and CDRH3) and three heavy chain complementarity determining regions (CDRL1, CDRL2 and CDRL3), wherein the set of CDRH1, CDRH2, CDRH3, CDRL1, CDRL2 and CDRL3 amino acid sequences is (a) SEQ ID NOs: 186, 187, 188, 190, 191 and 192; A combination of antibodies or pharmaceutical composition comprising said antibody; Said antibody for use in a method for treatment of a human or animal body by therapy; Said antibody for use in a method of treating or preventing a coronavirus infection, or a disease or complication associated with coronavirus infection; A method using said antibody for identifying the presence of coronavirus, or a protein or a protein fragment thereof, in a sample; A polynucleotide encoding said antibody; A vector or host cell comprising said polynucleotide; A method for producing said antibody comprising culturing said host cell

**1.2. claims: 1-15(partially)**

As for invention 1.1, but relying on a combination of HCDRs and LCDRs as recited in claim 1(b).

**1.3. claims: 1-15(partially)**

As for invention 1.1, but relying on a combination of HCDRs and LCDRs as recited in claim 1(c).

**1.4. claims: 1-15(partially)**

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

        As for invention 1.1, but relying on a combination of HCDRs and LCDRs as recited in claim 1(d).

1.5. claims: 1-15(partially)

        As for invention 1.1, but relying on a combination of HCDRs and LCDRs as recited in claim 1(e).

1.6. claims: 1-15(partially)

        As for invention 1.1, but relying on a combination of HCDRs and LCDRs as recited in claim 1(f).

1.7. claims: 1-15(partially)

        As for invention 1.1, but relying on a combination of HCDRs and LCDRs as recited in claim 1(g).

1.8. claims: 1-15(partially)

        As for invention 1.1, but relying on a combination of HCDRs and LCDRs as recited in claim 1(h).

1.9. claims: 1-15(partially)

        As for invention 1.1, but relying on a combination of HCDRs and LCDRs as recited in claim 1(i).

1.10. claims: 1-15(partially)

        As for invention 1.1, but relying on a combination of HCDRs and LCDRs as recited in claim 1(j).

1.11. claims: 1-15(partially)

        As for invention 1.1, but relying on a combination of HCDRs and LCDRs as recited in claim 1(k).

1.12. claims: 1-15(partially)

        As for invention 1.1, but relying on a combination of HCDRs and LCDRs as recited in claim 1(l).

1.13. claims: 1-15(partially)

        As for invention 1.1, but relying on a combination of HCDRs and LCDRs as recited in claim 1(m).

1.14. claims: 1-15(partially)

| | |
|---|---|
| Europäisches Patentamt<br>European Patent Office<br>Office européen des brevets | **LACK OF UNITY OF INVENTION**<br>**SHEET B** |

**Application Number**

**EP 22 21 3518**

The Search Division considers that the present European patent application does not comply with the requirements of unity of invention and relates to several inventions or groups of inventions, namely:

       As for invention 1.1, but relying on a combination of HCDRs and LCDRs as recited in claim 1(n).

1.15. claims: 1-15(partially)

       As for invention 1.1, but relying on a combination of HCDRs and LCDRs as recited in claim 1(o).

1.16. claims: 1-15(partially)

       As for invention 1.1, but relying on a combination of HCDRs and LCDRs as recited in claim 1(p).

             ---

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

EP 22 21 3518

This annex lists the patent family members relating to the patent documents cited in the above-mentioned European search report.
The members are as contained in the European Patent Office EDP file on
The European Patent Office is in no way liable for these particulars which are merely given for the purpose of information.

01-05-2023

| Patent document cited in search report | | Publication date | Patent family member(s) | | Publication date |
|---|---|---|---|---|---|
| WO 2022238481 | A1 | 17-11-2022 | NONE | | |
| US 2022267415 | A1 | 25-08-2022 | US 2022267415 A1 | | 25-08-2022 |
| | | | WO 2022177870 A1 | | 25-08-2022 |
| WO 2022184659 | A1 | 09-09-2022 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2022062777 W
- WO 9308829 A
- US 5731168 A
- WO 2009089004 A1
- US 4676980 A
- WO 2022238481 A

### Non-patent literature cited in the description

- **OU et al.** *Journal of Virology,* 2021, vol. 95 (16), e00617-22
- **STARR et al.** *Cell,* 2020, vol. 182, 1295-1310
- **YI et al.** *Cell. Mol. Immunol.,* 2020, vol. 17, 621-630
- **BAUM et al.** *Science,* 2020, vol. 369, 1014-1018
- **WANG et al.** *J. Virol.,* 2018, vol. 92
- **TER MEULEN et al.** *PLos Med.,* 2006, vol. 3, e237
- **KU et al.** *Nat. Commun.,* 2021, vol. 12, 469
- **ZOST et al.** *Nature,* 2020, vol. 584, 443-449
- **DU et al.** *Cell,* 2020, vol. 183, 1013-1023
- **WU et al.** *Science,* 2020, vol. 368, 1274-1278
- **ZOLA H.** Monoclonal Antibodies: a manual of techniques. CRC Press, 1987
- **HURRELL JGR.** Monoclonal Hybridoma Antibodies: techniques and applications. CRC Press, 1982
- **MILSTEIN ; CUELLO.** *Nature,* 1983, vol. 305, 537
- **TRAUNECKER et al.** *EMBOJ,* 1991, vol. 10, 3655
- **BRENNAN et al.** *Science,* 1985, vol. 229, 81
- **KOSTELNY et al.** *J. Immunol,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol,* 1994, vol. 152, 5368
- **TUTT et al.** *J. Immunol.,* 1991, vol. 147, 60
- **VERMA R et al.** *J. Immunol. Methods,* 1998, vol. 216, 165-181
- Current Protocols in Molecular Biology. Wiley Interscience, 1999
- Maniatis Manual. Cold Spring Harbor Publishing
- **DEVEREUX et al.** *Nucleic Acids Research,* 1984, vol. 12, 387-395
- **ALTSCHUL S. F.** *J Mol Evol,* 1993, vol. 36, 290-300
- **ALTSCHUL, S, F et al.** *J Mol Biol,* 1990, vol. 215, 403-10